(19) Europäisches Patentamt / European Patent Office / Office européen des brevets

(11) EP 1 130 998 B1

(12) **EUROPÄISCHE PATENTSCHRIFT**

(45) Veröffentlichungstag und Bekanntmachung des Hinweises auf die Patenterteilung:
**13.08.2008 Patentblatt 2008/33**

(21) Anmeldenummer: **99960985.2**

(22) Anmeldetag: **18.11.1999**

(51) Int Cl.:
***A61B 5/00*** *(2006.01)*

(86) Internationale Anmeldenummer:
**PCT/EP1999/008864**

(87) Internationale Veröffentlichungsnummer:
**WO 2000/028887 (25.05.2000 Gazette 2000/21)**

(54) **VORRICHTUNG ZUR NICHTINVASIVEN BESTIMMUNG DES SAUERSTOFFUMSATZES IN GEWEBEN**

DEVICE FOR NON-INVASIVELY DETECTING THE OXYGEN METABOLISM IN TISSUES

DISPOSITIF POUR LA DETERMINATION NON INVASIVE DU METABOLISME DE L'OXYGENE DANS DES TISSUS

(84) Benannte Vertragsstaaten:
**AT BE CH CY DE DK ES FI FR GB GR IE IT LI LU MC NL PT SE**

(30) Priorität: **18.11.1998 DE 19853028**

(43) Veröffentlichungstag der Anmeldung:
**12.09.2001 Patentblatt 2001/37**

(73) Patentinhaber: **LEA Medizintechnik GmbH**
**35394 Giessen (DE)**

(72) Erfinder: **Krug, Dr., Alfons**
**35444 Biebertal (DE)**

(74) Vertreter: **Müller, Eckhard**
**Mühlstrasse 9a**
**65597 Hünfelden-Dauborn (DE)**

(56) Entgegenhaltungen:

| | |
|---|---|
| **WO-A-96/39927** | **WO-A-96/41566** |
| **DE-A- 19 634 152** | **DE-A- 19 640 807** |
| **GB-A- 2 132 483** | **US-A- 5 131 391** |
| **US-A- 5 524 617** | **US-A- 5 772 587** |

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft eine Vorrichtung zur Ermittlung des lokalen Sauerstoffumsatzes und/oder des Sauerstoffverbrauchs und/oder der $O_2$-Transportkapazität und/oder der transportierten Sauerstoffmenge und/oder der Sauerstoffsverbrauchsrate und/oder der Sauerstoffumsatzrate in durchblutetem Gewebe und/oder aus dem Gehalt an Farbstoffen des durchbluteten Gewebes - ausgenommen Hämoglobin - abgeleiteter Daten gemäß dem Anspruch 1. Die Erfindung betrifft ferner ein Verfahren zur Ermittlung der vorgenannten Parameter gemäß dem Anspruch 16.

Technologischer Hintergrund und Stand der Technik

Der Sauerstoff ist von elementarer Bedeutung für fast alle Zellen in biologischen Geweben. In durchbluteten Geweben wird der Sauerstoff zum größten Teil in gebundener Form am Hämoglobin, das in den Erythrozyten eingelagert ist, von der Lunge zur den sauerstoffverbrauchenden Zellen transportiert. Arterielles Hämoglobin im großen Körperkreislauf, das in der Lunge aufgesättigt wurde, ist fast zu 100 % mit Sauerstoff gesättigt. Im peripheren Gewebe wird der Sauerstoff dann entlang der Kapillaren an die Zellen abgegeben. Entsprechend niedrigere Sättigungen des Hämoglobins mit Sauerstoff werden in den Gewebebereichen am venösen Ende der Kapillare und in den nachgeschalteten Venolen und Venen gemessen.

Die Messung des Sauerstoffgehalts läßt sich, dem Stand der Technik entsprechend, nur mit sehr aufwendigen Verfahren, wie NMR (Nuclear Magnetic Resonance), nicht invasiv im Gewebe durchführen. Zudem sind dynamische Messungen des Sauerstoffverbrauchs oder ein Monitoring der Sauerstoffentnahme durch die Zellatmung mit den NMR-Methoden bisher nicht möglich.

Der energetische Metabolismus der Zellen, der eng an die der Sauerstoffaufnahme gekoppelt ist, kann nur invasiv bestimmt werden, durch Entnahme von Gewebe- oder Körperflüssigkeitsproben und anschließender biochemischer Laboranalyse der Stoffwechselprodukte. Somit sind mit diesen biochemischen Labormethoden keine dynamischen Messungen des Sauerstoffverbrauchs oder ein Langzeitmonitoring möglich.

Die arterielle Sättigung des Hämoglobins $SO_2$ (%) im arteriellen Strombett kann durch sogenannte Pulsoximeter bestimmt werden, die jedoch herkömmlicherweise nicht die Bestimmung der kapillär-venösen Hämoglobin-Sättigungswerte, der Hämoglobinkonzentration im Meßvolumen oder der Blutflusses ermöglichen. Die bei der Pulsoximetrie verwendeten Verfahren basieren auf Erkenntnissen aus der Küvettenphotometrie und verwenden nur wenige Einzelwellenlängen, so daß eine Meßvolumenveränderung und die Registrierung von Streu- oder Absorptionsveränderungen nicht berücksichtigt werden können. Dadurch sind diese Meßverfahren mit erheblichen Meßunsicherheiten belastet. Der Vorteil dieser Geräte bisher ist, daß sie sehr günstig sind und leicht zu handhaben sind.

Es sind heute einige spektrometrische und spektroskopische Verfahren zur Bestimmung der Sättigung des Hämoglobins $SO_2$ [%] im kapillär-venösen Strombett und der Menge an Hämoglobin im Meßvolumen bekannt (EMPHO, NIRO500, HemoSpec und neu AbTisSpec). Diese Systeme erlauben zum Teil eine quantitative Bestimmung der Sättigung des Hämoglobins mit Sauerstoff $SO_2$ [%]. Im Vergleich hierzu ist die Bestimmung der Blutmenge oder der Hämoglobinkonzentration im kapillär-venösen Gewebebett mit den genannten Systemen nur in relativen Maßzahlen möglich. Mit den genannten Verfahren ist eine quantitative Angabe der Hämoglobinkonzentration im Meßvolumen bzw. der Blutmenge im Gewebe nicht möglich, da diesen Verfahren der Bezug auf den Detektionsbereich und damit auf den Lichtweg bzw. die Tiefenselektivität fehlt.

Einen möglicher Ansatz der Quantifizierung des Lichtweges und damit des effektiven Meßvolumens bieten zwei Verfahren; erstens die sogenannte PMS (Phase-Modulated Spektroscopie) und zweitens die TRS (Time-Resolved Spektroscopie). Diese zeitaufgelösten Verfahren haben jedoch den entscheidenden Nachteil, daß sie aufgrund des notwendigen Einsatzes von Laserquellen nur mit wenigen Wellenlängen arbeiten können. Somit ist die Bestimmung der Sättigung des Hämoglobins mit $O_2$, die aus der spektralen Absorptionsveränderung des Lichtes ermittelt wird, nur schwierig zu erreichen und wenn, dann nur mit sehr teueren Laserverfahren.

Mit Laser-Doppler-Verfahren, wie z. B. dem OptoFlow, lassen sich die Blutflußgeschwindigkeiten und die Blutflußmenge als relative Größen gewinnen. Diese Klasse von optischen Verfahren, die auf der Auswertung der Doppler-Signale basieren, ermöglichen keine Aussage zur Bestimmung der Sauerstoffbeladung der Erythrozyten (der roten Blutkörperchen) oder des aktuellen Meßvolumens.

Für eine vollständige Beschreibung des Zustandes der Sauerstoffversorgung von durchbluteten Geweben müssen jedoch zusätzlich zu dem Parameter der Blutflußgeschwindigkeit auch die bewegte Blutmenge (Anzahl der bewegten roten Blutkörperchen), die gesamte Blutmenge im Gewebe (auch Hämoglobinkonzentration genannt) und die Beladung der roten Blutkörperchen mit Sauerstoff (auch Hämoglobinoxygenierung oder Sättigung $SO_2$ genannt) meßtechnisch bestimmt werden.

Aus der EP 0 353 619 A ist eine Vorrichtung und ein Verfahren zur Bestimmung von Sauerstoffgehalt und Gewebefarbstoffen bekannt, wobei Licht mittels Lichtleitfasern von einer Lichtquelle zu einem Sensor und rückgestreutes Licht über Lichtleitfasern zu Detektoren geführt wird. Als Lichtquelle ist eine Weißlichtquelle vorgesehen.

Aus der WO 96/39 927 A ist dagegen zur Bestimmung verschiedener Körpereigenschaften wie Hämoglobin- oder Sauerstoffgehalt vorgesehen, dass die Vorrichtung hierzu eine Laserquelle aufweist.

Aus der Dissertation "Quantitative optische Gewebemessungen am Herzen und an der Leber" des Erfinders vom 24.2.1998 ist aus Abbildung 2.4-1 eine Messanordnung bekannt, mittels welcher in einer Kuvette invitro vergleichende Messungen mittels zweier Messverfahren durchgeführt werden.

Aus der EP 771 546 A2 ist eine Vorrichtung zur nicht invasiven optischen Messung des Blutflusses in biologischem Gewebe bekannt.

Die Lichtausbreitung in biologischen Medien, Streususpensionen, Gewebeschnitten und intakten Geweben und Zellstrukturen wird bestimmt durch deren optische Eigenschaften. Die Lichtausbreitung wird dabei durch die beiden Grundphänomene Lichtabsorption und Lichtstreuung bestimmt. Die Absorption des Lichtes oder die Abschwächung im Sinne einer Umwandlung der Lichtenergie in Licht einer höheren Wellenlänge, findet durch Wechselwirkung mit zellulären und subzellulären Strukturen von Makromolekülen und Einzelmolekülen statt. Ein stark absorbierendes Molekül im sichtbaren Bereich der Wellenlängen ist z. B. die Häm-Gruppe im Hämoglobinmolekül. Durch Absorption verliert sichtbares Licht einen Teil seiner Intensität auf dem Lichtweg durch das Gewebe. Die Absorptionsmessungen der Erfindung beziehen sich primär auf das Hämoglobin, den roten Blutfarbstoff, der in nahezu allen Geweben auch der stärkste Absorber im sichtbaren Wellenlängenbereich ist.

Im Unterschied hierzu verliert das Licht durch elastische Streuung keine Energie. Die einlaufende elektromagnetische Welle, die mit dem Streuzentrum wechselwirkt, strahlt nach der Anregung durch die einlaufende Lichtwelle die Energie wieder in die verschiedenen Raumwinkel ab. Dabei behält das Licht seine Wellenlänge bei, nur die Ausbreitungsrichtung des Lichtes wird durch die Streuzentren verändert. Der physikalische Vorgang auf molekularer Ebene ist so vorzustellen, daß die Streupartikel durch die einlaufende Lichtwelle angeregt werden und daraufhin die Energie bei derselben Wellenlänge wieder an den Raum abgeben. Es hängt nun von der Geometrie, der Form und der elektromagnetischen Verteilung der Elektronenschalen des Moleküls sowie der Struktur ab, in welche Richtungen eine Abstrahlung der Lichtenergie stattfindet.

Der einfallende, gerichtete Strahl wandelt sich auf dem Weg durch das Gewebe um in diffuse Strahlung und addiert sich zur Vorwärtsstreuung der angeregten Streuzentren. Nach unter Umständen mehrfacher Streuung gelangt ein kleiner Teil der eingestrahlten Lichtintensität zur Oberfläche zurück. Die Rückwärtsabstrahlung aller Streuzentren summiert sich zur Rückwärtsstreuung. Mit den Lichtleitern an der organoberfläche kann nur Licht detektiert werden, das rückgestreut und auf seinem Lichtweg durch Absorption nicht ausgelöscht wurde. Der Lichtleiter erfasst jedoch an der Organoberfläche nur Licht, das innerhalb der Lichtleiterapertur rückgestreut wird. Diese Vorstellung von der Lichtausbreitung ist formal beschrieben in der Strahlungstransporttheorie.

Aufgabenstellung

Aufgabe der Erfindung ist die meßtechnische Erfassung der Hämoglobinoxygenierung und der Hämoglobinkonzentration in durchblutetem Gewebe sowie davon abgeleiteter Meßwerte, sowie der Vorschlag eines Gerätes zur Ermittlung des Sauerstoffgehalts und anderer verwandter Parameter (siehe Tabelle 1) in durchblutetem Gewebe.

Erfindung und vorteilhafte Wirkungen

Diese Aufgabe wird erfindungsgemäß gelöst von einer Vorrichtung mit den Merkmalen des Anspruchs 1 oder 15 sowie von einem Verfahren mit den Merkmalen des Anspruchs 16. Ausführungen der Erfindung sind Gegenstände von Unteransprüchen. Ein geeigneter Name für das erfindungsgemäße Gerät wäre AbTisSpec, ein Akronym für Absorptions Tissue Spectrophotometer.

Erfindungsgemäß wird also eine Vorrichtung sowie ein Verfahren vorgeschlagen, welche bzw. welches den Sauerstoffgehalt sowie davon abgeleitete Daten mit einem optischen Sensor zum Auflegen auf das durchblutete Gewebe erfaßt. Es sind mehrere Lichtquellen vorgesehen, die Licht über Lichtleitfasern zum Sensor schicken, einen oder mehrere Detektoren, die vom Gewebe zurückgestreutes Licht über Lichtleitfasern empfangen und eine Auswerteeinheit, die aus dem rückgestreuten Licht Erkenntnisse über den Sauerstoffhaushalt gewinnt. Als Lichtquellen werden eine Weißlichtquelle und eine Laserquelle verwendet. Während die Weißlichtquelle das Licht ins Gewebe einbringt, dessen rückgestreute Anteile später spektral zerlegt werden, bringt die Laserlichtquelle ein monochromatisches Licht ein, an dessen rückgestreutem Anteil eine Frequenzverschiebung meßbar ist, so daß eine Dopplermessung und damit eine Geschwindigkeitsmessung möglich sind.

Für die Illumination wird eine Weißlichtquelle (oder verschiedene breitbandige LED's) verwendet. Entscheidend ist eine Lichtquelle mit hoher Leuchtfelddichte und einem möglichst weißen und glatten Spektrum. Das aus dem Gewebe rückgestreute Licht wird über einen Polychromator spektral zerlegt, verstärkt und nachfolgend als wellenlängenabhängiges Licht-Intensitätsmuster zur Auswertung gebracht.

Der Wellenlängenbereich von 500 bis 650 nm (VIS) ist besonders für oberflächennahe Messungen geeignet.

Dagegen ist der Wellenlängenbereich von 600 nm bis 900 nm (NIR) besonders für tiefenselektive Messungen auch in größeren Tiefen geeignet, da die effektive Eindringtiefe des Lichtes im NIR Wellenlängenintervall größer ist als im zuerst genannten Wellenlängenintervall. Durch verschiede Abstände von Illuminations- und Detektionsbereichen werden zudem unterschiedliche Meßvolumina durch die Sensorgeometrie vorgegeben. Die Kombination der Auswahl von Detektionsabständen und entsprechenden Wellenlängenintervallen kann somit klar definiert werden und erlaubt die deutliche Abgrenzung einzelner Meßvolumina gegeneinander. Für die Berechnung der Sättigungswerte wird die spektrale Form der Spektren, z. B. über ein Formerkennungs- und Mischverfahren oder einer Approximation der Diffusionsgleichung, ausgewertet. Als Meßwerte erhält man erfindungsgemäß tiefenselektiv die Sättigung des Hämoglobins mit $O_2$ im kapillärvenösen Gewebebett.

Das Meßvolumen wird durch ein neues Verfahren, nämlich über die Messung eines Intensitätsgradienten an der Oberfläche in Kombination mit der Bestimmung der Absorptions- und Streukoeffizienten aus den Dopplermessungen, sowie der spektrometrischen Messungen, bestimmt.

Die Hämoglobinmenge läßt sich aus der Auswertung der spektrometrischen Daten gewinnen. Die Absorption hervorgerufen durch die Lichtabschwächung durch das Hämoglobin wird über ein Verfahren bestimmt, dessen Grundzüge erstmals bei A. Krug, Dissertation, Uni Erlangen-Nürnberg 1998, beschrieben werden.

Für die Beschreibung der vollständigen. Sauerstoffversorgungssituation bedarf es einer Vielzahl von Meßgrößen (siehe Tabelle 1). Es muß die arterielle Sättigung und unabhängig hiervon die kapillar-venöse Sättigung des Hämoglobins bestimmt werden. Aus der Differenz von arterieller Sättigung zur Sättigung des Hämoglobins in den Kapillaren, Venolen und Venen läßt sich dann der $O_2$-Umsatz, bzw. die Sauerstoffaufnahme bestimmen. Verrechnet man diese Differenzmenge mit den Blutflußwerten, so ergibt sich hieraus der $O_2$-Verbrauch im untersuchten Gewebevolumen, der somit lokal bestimmt werden kann.

Zusammengefaßt müssen folgende Bestimmungsgrößen für die Bestimmung der lokalen Sauerstoffversorgung ermittelt werden:

1. Tiefenselektiv die Sättigung des Hämoglobins $SO_2$ [%] im arteriellen Strombett.
2. Tiefenselektiv die Sättigung des Hämoglobins $SO_2$ [%] im kapillär-venösen Strombett.
3. Tiefenselektiv die Menge an Hämoglobin im Meßvolumen, aus den Absorptionskoeffizienten.
4. Größe des Meßvolumens, resultierend aus der Oberflächengradientenmessung, den Streukoeffizienten und den Anisotropiefaktoren, oder aus Modellen der Diffusionstheorie.
5. Tiefenselektiv die Blutflußmenge und die Blutflußgeschwindigkeit.
6. Die lokale Gewebetemperatur.

Die Kombination dieser Meßgrößen und die Detektion über einen integrierenden Sensor, der Messungen in dem gleichen Areal garantiert, erlaubt eine Messung des lokalen Sauerstoffumsatzes von durchblutetem Gewebe.

Als Auswerteeinheit können ein Spektrometer, ein Spektroskop, ein Laser-Doppler-Spektroskop, ein Gewebespektrometer, ein Gewebespektroskop, ein Pulsoximeter und/oder ein Temperaturmeßgerät je allein oder in beliebiger Kombination vorgesehen sein.

Damit lasser sich

a) der lokale Sauerstoffgehalt,
b) der lokale Sauerstoffverbrauch in arteriell-venös gemischtem Gewebe,
c) die Sauerstoffverbrauchsrate in arteriell-venös gemischtem Gewebe,
d) die gesamte Blutmenge,
e) die lokale Sauerstofftransportkapazität,
f) die lokal transportierte Sauerstoffmenge,
g) der Sauerstoffumsatz in arteriell-venös gemischtem Gewebe,
h) die Sauerstoffumsatzrate in arteriell-venös gemischtem Gewebe und/oder
i) der lokale Gewebe-Sauerstoffpartialdruck in durchbluteten Geweben ermitteln.

In einer Ausführung der Erfindung werden durch Auswahl des Wellenlängenbereichs und der Detektor-Sender-Separation Informationen aus unterschiedlichen Tiefen gewonnen. So können Meßwerte sowohl von der Gewebeoberfläche als auch aus tieferen Regionen ermittelt werden.

Die erfindungsgemäße Vorrichtung eignet sich nicht nur zur Detektion des Hämoglobins und der daraus ableitbaren Meßwerte, sondern auch zur Ermittlung des Gehalts an Gewebefarbstoffen, wie Cytochrome, Myoglobin, Melanin, Bilirubin oder anderen im Gewebe anwesenden Farbstoffen, sowie davon abgeleiteter Daten. Dazu kann der gleiche optische Sensor zum Auflegen auf das Gewebe verwendet werden. Ebenso die eine oder die mehreren Lichtquellen, die Licht über Lichtleitfasern zum Sensor schicken. Zudem ein oder mehrere Detektoren, die vom Gewebe zurückgestreutes Licht über Lichtleitfasern empfangen und einer Auswerteeinheit zuleiten. Die Auswerteeinheit verarbeitet die empfangenen Daten entsprechend und ermittelt den Farbstoffgehalt, dessen Verteilung oder dessen Transport.

Auch in dieser Vorrichtung ist als Lichtquelle eine Weißlichtquelle und eine Laserquelle vorgesehen.

Ebenso können als Auswerteeinheit ein Spektrometer, ein Spektroskop, ein Laser-Doppler-Spektroskop, ein Gewebespektrometer, ein Gewebespektroskop, ein Pulsoximeter und/oder ein Temperatursensor je einzeln oder in beliebiger Kombination vorgesehen sein.

Durch die Auswahl des Wellenlängenbereichs und der Detektor-Sender-Separation können Informationen aus unterschiedlichen Tiefen gewonnen werden.

In einer weiteren Ausführung der Erfindung kann ein Bündel an Lichtleitfasern, das vom Sensor zum Detektor oder zu einer Kamera, wie einer CCD-Kamera, reicht, vorgesehen sein, so daß eine zweidimensionale Abbildung der aufgenommenen Meßwerte erzeugt werden kann. Damit kann eine äußerst anschauliche Abbildung der flächigen Hämoglobinverteilung und/oder der Sauerstoffsättigung und/oder der Sauerstoffparameter, entsprechend Tabelle 1, und/oder der Verteilung anderer Farbstoffe in oder am Rand eines Gewebes hergestellt werden.

Bei Verwendung eines zusätzlich tiefenselektiven Sensors oder einer tiefenselektiven Auswertung kann eine dreidimensionale

Abbildung der aufgenommenen Meßwerte erzeugt werden. Dies erlaubt einen "Einblick" ins Innere eines Organs oder eines Gewebes und erlaubt eine schichtweise Darstellung der relevanten Daten.

Entscheidend für diese optischen Messungen entsprechend der Erfindung ist der direkte Kontakt des Sensors mit der Organoberfläche. Nur hierdurch ist es möglich, Licht-Rückstreumessungen anstelle von z.B. Licht-Reflexionsmessungen durchzuführen. Nur bei einem aufliegenden Sensor können tiefenselektive Messungen oder auch meßvolumenbezogene Messungen durchgeführt werden. Für Messungen der Mikrozirkulation ist es natürlich entscheidend, entsprechende mechanische Applikatoren zu konstruieren, die die Mikrozirkulation auch in der oberflächennahen Kapillarschicht nicht beeinträchtigen.

Ausführungsbeispiele

Weitere Vorteile und Anwendungen der vorliegenden Erfindung ergeben sich aus der nachfolgenden Beschreibung mehrerer Ausführungsbeispiele anhand der Zeichnungen.

Es zeigen:

Fig. 1 einen integrierten Sensor,
Fig. 2 Applikationsform des Sensor, Integration von Laser-Doppler und Gewebespektrometer,
Fig. 3 Applikationsform des Sensor, Integration von Laser-Doppler und Gewebespektrometer,
Fig. 4 Applikationsform des Sensor, Integration von Gewebespektrometer und/oder Laser-Doppler,
Fig. 5 Applikationsform des Sensor, Integration von Gewebespektrometer und/oder Laser-Doppler,
Fig. 6 Applikationsform des Sensor, Integration von Laser-Doppler und Gewebespektrometer,
Fig. 7 ein Gewebespektrometer,
Fig. 8 ein Zweischichtenmodell,
Fig. 9 Gewebe-Rohspektren,
Fig. 10 Hämoglobinspektren 0 bis 100 % oxygeniert,
Fig. 11 einen Ohrsensor,
Fig. 12 einen Secure-Sensor,
Fig. 13 die Extinktion als Funktion der Hämoglobinkonzentration und Oxygenierung,
Fig. 14 die Extinktion als Funktion der Hämoglobinkonzentration im isoliert perfundierten Gewebe,
Fig. 15 Rückstreufunktionen in x- und z-Richtung
Fig. 16 eine Streuküvette,
Fig. 17 Detektionstiefen,
Fig. 18 Übertragungsfunktion der x- zu z-Gradienten,
Fig. 19 das Modell eines Meßvolumens,
Fig. 20 zwei Absorptionsspektren,
Fig. 21 eine Vorrichtung zur Aufnahme zweidimensionaler Meßwertverteilungen,
Fig. 22 eine zweidimensionale Meßwertverteilung,
Fig. 23 eine Vorrichtung zur Aufnahme dreidimensionaler Meßwertverteilungen und
Fig. 24 Cytochromspektren, oxidierte Cytochrome und reduzierte Cytochrome gemessen in Mitochondrien-Suspension.

Fig. 1 zeigt schematisch von unten, also vom Gewebe aus, gesehen, eine lediglich beispielhafte Ausführung eines integrierten Sensor S oder Meßkopfes, wie er in der erfindungsgemäßen Vorrichtung Verwendung finden kann. Er enthält hier zur Illumination die Weißlichtquelle W, die Laserlichtquelle L und eine Mehrzahl an Detektoren, wobei mit DD die Detektoren für Dopplersignale und mit DR die Detektoren für rückgestreute Intensitäten bezeichnet sind. Die gezeigte linienförmige Anordnung ist lediglich beispielhaft. Viele andere Anordnungen sind möglich, wobei die Detektoren DR, die weiter von der Lichtquelle W entfernt sind, Licht empfangen, das in tiefere Regionen des Gewebes eingedrungen ist und dort rückgestreut wurde, wie in der EP 771 546 A2 beschrieben ist. Zusätzlich ist ein Temperatursensor DT gezeigt.

Erfindungsgemäß werden über das integrierte Sensorsystem S Photonen einer kohärenten monochromatischen Lichtquelle L und bevorzugt zusätzlich Photonen einer/mehrerer breitbandiger Weißlichtquellen W in das Gewebe in einem ersten Bereich eingestrahlt. In unterschiedlichen Abständen zu diesem ersten Bereich werden die wieder austretenden Photonen detektiert (DD, DR). Im räumlichen Wechsel wird das Licht detektiert für die Laser-Doppler-Auswertung und für die spektroskopische oder spektrometrische Auswertung. Der Aufbau des integrierten Sensors ist in Fig. 1 beispielhaft dargestellt.

Die tiefenselektiven Laser-Doppler-Messungen können mit einer Vorrichtung, wie sie in der EP 771 546 A2 beschrieben ist, durchgeführt werden.

Erfindungsgemäß wird in Fig. 2 eine spezielle Form des Applikators gezeigt, die besonders für endoskopische Messungen im Magen-Darmbereich Anwendung findet. Der Vorteil dieser Faseranordnung liegt in der kompakten Bauform und leicht reproduzierbaren Geometrie. Wie oben steht W für die Stelle der Beleuchtung mit einer Weißlichtquelle, L für die Beleuchtung mit der Laserquelle. Im räumlichen Wechsel wird das Licht detektiert für die Laser-Doppler-Auswertung DD und für die spektroskopische oder spektrometrische Auswertung DR. Dieser Sensor erlaubt Messungen in jeweils zwei Detektionstiefen. Der Temperatursensor DT kann optional im Zentrum des Sensors angeordnet werden, oder eine weitere Faser.

Fig. 3 zeigt eine erfindungsgemäß leicht veränderte Anordnung aus Fig. 2, für Anwendungen mit zwei Separationen für die Laser-Doppler-Messungen DD und Auswertung DR der spektrometrischen und/oder spektroskopischen Messungen in einer Separation, jedoch mit größeren Gesamtquerschnitten, aufgrund der Zusammenfassung der Intensitäten dreier Detektionsfasern. Fig. 2 zeigt eine Weißlichtquelle W und eine Laserlichtquelle L.

Die Faseranordnung der Fig. 4, zeigt eine erfindungsgemäße Vorrichtung zur Bestimmung der Primärsignale die über ein Gewebespektrometer gewonnen werden, der arteriellen Sauerstoffsättigung, der lokalen Sauerstoffsättigung des Hämoglobins, sowie der lokalen Hämoglobinkonzentration, wie auch der Parameter aus Tabelle 3. Diese Anordnung hat aufgrund der homogenen Ausleuchtung des zentralen Areals durch die hier beispielhaft.gezzeigten sechs Weißlichtquellen W besondere Vorteile auch als Reflexionssensor eingesetzt werden zu können. Diese Vorrichtung ist dadurch gekennzeichnet, daß sich die Fasern W zur Beleuchtung auf einem Kreis um die Detektorfaser DR befinden. In diese Fasern W wird über eine oder mehrere Lichtquellen Licht zum Sensor S transportiert.

Fig. 5 zeigt eine erfindungsgemäß veränderte Form der in Fig. 4 gezeigten Vorrichtung für die gleichen, dort genannten Anwendungen, nur für Messungen in größeren Eindringtiefen. Die größeren Eindringtiefen werden aufgrund der größeren Sender-Detektor-Separation erreicht. Auch lassen sich beide Vorrichtungen (Fig. 5 und Fig. 4) kombinieren, dann wenn die Beleuchtungsquellen auf einer Kreisanordnung alternierend in Bezug auf Zeit oder Wellenlänge benutzt werden.

Fig. 6 ist ein veränderte Form des Basissensors aus Fig. 1, in der Art, daß anstelle von jeweils einer Faser in jeder Separation eine ganze Linie, bestehend aus mindestens 2 Fasern angeordnet wurde. Dieser Sensor erlaubt integrierende Messungen in größeren Arealen. Die Tiefenselektivität des Sensors aus Fig. 1 bleibt nahezu erhalten. Vorrichtung dadurch gekennzeichnet, daß die Fasern einer Separation (xi) parallel ausgewertet werden.

Die Fig. 7 gibt den prinzipiellen Aufbau eines Gewebephotometers oder Gewebespektrometers wieder. Die Kernkomponenten sind eine breitbandige Lichtquelle W, zum Sensor S führende Lichtleitfasern für die Illumination des Gewebes und vom Sensor S wegführende Lichtleitfasern für die spektral-zerlegte Detektion des rückgestreuten Lichtes aus dem Gewebe. Die Detektionseinheit enthält einen Polychromator, der zugleich das Licht spektral zerlegt und wellenlängenabhängig die detektierten Intensitäten quantifiziert. Somit stellt das Gewebephotometer als Ausgangswerte die Farbspektren zur Verfügung, die für die spezifischen, sich anschließenden Auswertungen der spektralen Information verwendet werden. Im Aufbau nach Fig. 7 wurde parallel zum Polychromator noch eine spektroskopische Empfangseinheit geschaltet, die es erlaubt, in einem eingeschränkten Wellenlängenbereich bzw. bei Einzelwellenlängen die detektierten Rückstreuintensitäten mit größerer Geschwindigkeit und höherer Empfindlichkeit erfassen zu können. Wichtig wird diese Detektoreinheit für die Auswertung der pulsatilen Blutsignale, so z. B. bei der Bestimmung der arteriellen Sättigung des Hämoglobins.

Für die Illumination wird zum Beispiel eine Weißlichtquelle W (oder verschiedene breitbandige LED's) verwendet. Entscheidend ist eine Lichtquelle mit hoher Leuchtfelddichte und einem möglichst weißem und glatten Spektrum. Das aus dem Gewebe rückgestreute Licht wird - spektral zerlegt über einen Polychromator - verstärkt und nachfolgend als wellenlängenabhängiges Licht-Intensitätsmuster zur Auswertung gebracht.

Für die Detektion der oberflächennahen Hämoglobinwerte sind Detektoreinheiten notwendig, die besonders hohe Empfindlichkeit im sichtbaren Wellenlängenbereich besitzen. Die Absorptionswerte des Hämoglobins erlauben im Wellenlängenband von 500 bis 650 nm die Bestimmung der Hämoglobinwerte bis in eine Tiefe von maximal 4 Millimetern.

Entsprechend der Erfindung werden zwei Wellenlängenbereiche definiert und unterschieden.

Der Wellenlängenbereich von 500 bis 650 nm (VIS) ist besonders für oberflächennahe Messungen und der Wellenlängenbereich von 580 nm bis 900 nm (NIR) ist besonders für tiefenselektive Messungen auch in größeren Tiefen und größeren Volumina geeignet, da die effektive Eindringtiefe des Lichtes im NIR Wellenlängenintervall größer als im zuerst genannten Wellenlängenintervall ist. Für die Detektion der Hämoglobinwerte im Makrovolumen ist eine Detektoreinheit notwendig, die besonders hohe Empfindlichkeit im infrarotnahen Wellenlängenbereich besitzt. Die Absorptionswerte des Hämoglobins im Wellenlängenbereich von 600 bis 900 nm sind um den Faktor 10 bis 20 niedriger als im sichtbaren Wellenlängenbereich. Durch die verminderte Lichtabschwächung sind prinzipiell höhere effektive Eindringtiefen in das Gewebe möglich. Durch verschiede Abstände von Illuminations- und Detektionsbereichen werden zudem unterschiedliche Meßvolumina durch die Sensorgeometrie vorgegeben. Die Kombination der Auswahl von Detektionsabständen und entsprechenden Wellenlängenintervallen kann somit klar definiert werden und erlaubt die deutliche Abgrenzung einzelner Meßvolumina gegeneinander.

Erfindungsgemäß sind erstens durch die Kombination der Auswahl des Wellenlängenbereichs von 500 bis 650 nm und der Lichtleiterseparation kleiner 2 mm selektiv oberflächennahe Messungen möglich. Zweitens sind durch die Auswahl des Wellenlängenbereichs von 650 bis 900 nm zusammen mit Lichtleitersepartionen größer 2 mm Messungen im Makrovolumen und in großen Detektionstiefen möglich.

Die Detektoreinheit mit Polychromator ist das Herzstück des erfindungsgemäßen Gewebespektrometers. Eine möglichst hohe Quantenausbeute und die daraus resultierende hohe Detektionsfrequenz sind erwünscht. Die höchsten Anforderungen an die Detektionsgeschwindigkeit werden bei der Applikation der Methode in der Kardiologie gestellt, da hier die schnellsten physiologischen Reaktionen zu erwarten sind. Die Sättigung des Hämoglobins $SO_2$ pulsiert am Myocard mit der Herzfrequenz. Die kritischen $SO_2$-Werte sind am Ende der Systole zu erwarten, da während der Kontraktion die Myocarddurchblutung, aufgrund des hohen Druckes im Ventrikel, stark eingeschränkt ist. Die Herzfrequenz beträgt ungefähr 1/sec, mit einer systolischen Kontraktionsdauer im Bereich von 100 ms. Daraus resultiert eine maximal notwendige Abtastfrequenz von 10 msec um dieses Intervall noch ausreichend auflösen zu können. Alle anderen physiologischen Vorgänge im menschlichen Organismus verlaufen entsprechend langsamer und können mit niedrigeren Abtastfrequenzen detektiert werden. Ein weiterer Vorteil hoher Abtastraten ist die steigende Bediensicherheit, die "verwacklungssichere" Aufnahmen gewährleisten.

Die nachfolgende Tabelle stellt eine Übersicht über die Sauerstoffparameter zur Beschreibung der lokalen Versorgungssituation dar, die mit dem erfindungsgemäßen integrierten Sensorkonzept möglich sind. In der Tabelle wurde eine Kreuz an die Stelle gesetzt, an der ein Signal entweder aus den Gewebespektrometerwerten, den Laser-Doppler-Daten den Pulsoximeterwerten oder aus den Temperaturwerten benötigt wird, um hieraus den entsprechenden Sauerstoffparameter bestimmen zu können. Die Definitionen der genannten lokalen Sauerstoffversorgungsgrößen werden untenstehend näher erläutert.

Tabelle 1: Zusammenstellung der Verfahren, durch die mit Einsatz des Gewebespektrometers, des Laser-Doppler-Spektroskops, des Pulsoximeters und/oder eines Temperaturmeßgerätes sich die klinisch relevanten Blut- bzw. Sauerstoffversorgungsgrößen bei Integration oder partieller Integration der Sensoren in einen gemeinsamen Meßkopf und integrativer Datenanalyse bestimmen lassen.

| Sauerstoffparameter | Gewebespektrometer | | | | Laser-Doppler | | | Pulsoximeter | Temp. |
|---|---|---|---|---|---|---|---|---|---|
| | $SO_2$ kap.-ven. | $Hb_{conc}$ | Meßvol. bzw. intensitäts gradient | $Hb_{conc/Vol}$ | Blood -Flow | Velocity v | Pulsatilität | $SO_2$ arteriell | T |
| Sauerstoffgehalt | X | | x | x | | | | | |
| Sauerstoffverbrauch | X | | x | x | | | x | x | |
| Blutmenge-Gesamt | | | x | x | | | | | |
| $O_2$-Transport-Kapazität | | x | | | x | | | | |
| Transp. $O_2$-Menge | x | x | | | x | | | | |
| Sauerstoffverbrauchs-rate | x | x | | | | | x | x | |
| Sauerstoffumsatz | x | | x | x | | x | x | x | |
| Sauerstoffumsatzrate | x | x | | | x | | x | x | |
| Gewebe-$pO_2$ | x | | | | | | | | x |

Nachfolgend werden die verschiedenen Sauerstoffparameter hergeleitet und die formalen Zusammenhänge der erfindungsgemäßen Verfahren definiert.

Eine Messung des Sauerstoffgehaltes im Blut setzt die Bestimmung der Hämoglobinmenge im untersuchten Gewebevolumen und die Bestimmung der Sättigung des vorhanden Hämoglobins voraus. Es ist deshalb unerläßlich, auch das illuminierte Gewebevolumen quantitativ bestimmen zu können, da die Hämoglobinmenge auf den Volumenwert bezogen werden muß. Die Größe des Illuminations- bzw. Meßvolumens wird maßgeblich bestimmt durch die Sensorgeometrie und die physikalischoptischen Grundparameter des Gewebes, die in Form von Absorptionskoeffizienten $\mu_a(\lambda)$ und Streukoeffizienten $\mu_s(\lambda)$ im verwendeten Spektralbereich der Lichtquellen W und L formuliert werden. Nachfolgend werden die angesprochenen Zusammenhänge in formalen Bezügen in Form von mathematischen Formeln schrittweise abgeleitet:

Der $O_2$-Gehalt im Blut läßt sich somit formal nach folgender Formel bestimmen:

$$O_{2\ Gehalt} = Hb_{Menge} \cdot SO_2 \cdot H$$

Somit gliedert sich das Meßproblem in zwei unterschiedliche Aufgabenstellungen. Einerseits in der Bestimmung der Hämoglobinmenge $Hb_{Menge}$ und andererseits in der Bestimmung der Sättigung des Hämoglobins $SO_2$ mit Sauerstoff. Die Hüfnersche Zahl *H* stellt den Zusammenhang zwischen Hämoglobingehalt und maximalem Sauerstoffgehalt her. Die Berechnung der Hämoglobinmenge wiederum wird in einem nachfolgenden Absatz beschrieben.

Der Sauerstoffumsatz bzw. der Sauerstoffverbrauch im Gewebe läßt sich formal beschreiben durch:

$$O_{2\ Verbrauch} = O_{2\ Gehalt\ arteriell} - O_{2\ Gehalt\ venös}$$

Mit der Annahme, daß die Blutmenge arteriell und venös aufgrund der Kontinuitätsgleichung dieselbe bleibt, läßt sich die Formel vereinfachen zu:

$$O_{2\ Verbrauch} = H \cdot \left(Hb_{Sättigung\ arteriell} - Hb_{Sättigung\ venös}\right) \bullet Hb_{Menge(im\ Meßvolumen)}$$

Aus der Literatur ist bekannt, daß das arterielle Blutvolumen im Gewebe typischerweise kleiner 5 % ist. Somit reicht es aus, nur die Hämoglobinmenge im kapillar-venösen Strombett zu bestimmen, ohne große Verfälschungen zu verursachen.

Beschränkt man sich anstelle der Bestimmung der Menge an $O_2$, die verbraucht wurde, auf die Sauerstoffverbrauchsrate, einer Verhältnisangabe von arteriellem zu venösem Verbrauch, so folgt hieraus folgende Bestimmungsgleichung:

$$O_{2\ Verbrauchsrate} = \left(SO_{2\ arteriell} - SO_{2\ venös}\right) \bullet Hb_{conc.}$$

Die $Hb_{conc.}$ ist in gleicher Weise zu bestimmen, wie unten definiert. Aus nachstehenden Erklärungen geht hervor, daß die $Hb_{conc.}$ proportional ist zum Extinktionswert (oder auch der Optical-Density) bezogen auf das jeweilige Meßvolumen.

Im Gewebe ist es nicht immer möglich, die gemessene arterielle Sättigung mit der zugehörigen kapillar-venösen Hämoglobinsättigung zu verrechnen. Das ist streng genommen nur dann der Fall, wenn die Kontinuitätsgleichung im entsprechenden Gewebevolumen erfüllt ist. Wichtig ist somit, daß sich im betrachteten Meßvolumen arterielle und kapillärvenöse Strombahnen vorhanden sind und der arterielle Puls zu detektieren ist.

Es kann jedoch lokal für jedes Gewebevolumen ein Größe bestimmt werden, die als transportierte Sauerstoffmenge oder lokale Sauerstofftransportkapazität bezeichnet werden soll. Diese Größen können mit dem neuen, integrierten Sensorkonzept erfaßt werden.

Die lokale Sauerstofftransportkapazität bestimmt sich aus der lokal vorhanden Zahl von bewegten Erythrozyten und deren Hämoglobingehalt, die ausgedrückt wird in der lokal bewegten Hämoglobinkonzentration $Hb_{conc}$, multipliziert mit der Hüfnerschen Zahl *H* und multipliziert mit der Fließgeschwindigkeit $v_{Blut}$ der Erythrozyten im untersuchten Areal. Laser-Doppler-Geräte, die eine spektrale Auflösung der Geschwindigkeit der Erythrozyten auflösen, liefern auch ein Signal, das die Menge der bewegten Erythrozyten widerspiegelt (MengeErys, bewegt)

Da die Laser-Doppler Geräte einen Wert berechnen, der einer relativen Durchblutung *Blood-Flow* entspricht, kann in einer Näherungslösung für relative Werte auch folgende Gleichung herangezogen werden. Der Blood-Flow gibt dabei ein Maß an für die Anzahl der bewegten Erythrozyten, multipliziert mit deren Geschwindigkeit, und stellt damit ein Maß für einen Volumenstrom dar. Damit ergibt sich:

$$O_{2\,Transportkap.} = \frac{H \cdot Hb_{conc} \cdot BloodFlow}{Menge_{Erys.bewegt}}$$

Die lokal transportierte Sauerstoffmenge bestimmt sich aus der lokal vorhandenen Zahl von Erythrozyten, multipliziert mit der Flußgeschwindigkeit der Erythrozyten. Dieses Produkt wird von den Laser-Doppler Geräten als ein Wert berechnet, des als Blood-Flow bezeichnet wird. Blood-Flow multipliziert mit der Hüfnerschen Zahl und der lokalen Sauerstoffsättigung des Hämoglobins ergibt die Transportierte Sauerstoffmenge im untersuchten Areal.

$$Transportierte\ O_{2Menge\ rel.} = H \cdot SO_2 \cdot BloodFlow$$

Der Sauerstoffumsatz ist der Menge an $O_2$, die in einem bestimmten Gewebeareal verbraucht wird, proportional. Die verbrauchte $O_2$-Menge ergibt sich aus der Differenz der Sauerstoffmenge, die in das Gewebe transportiert wird (arteriell), gegenüber der Menge, die auf venöser Seite wieder abtransportiert wird. Die umgesetzte Sauerstoffmenge läßt sich, wie gezeigt, berechnen zu:

$$O_{2_{Umsatz}} = (SO_{2_{arteriell}} - SO_{2_{venös}}) \cdot \frac{H \cdot Hb_{Menge} \cdot v_{Blut} \cdot Menge_{Erys.bewegt}}{im\,Meßvolumen}$$

Es gelten hierbei die gleichen Annahmen, Näherungen und Abkürzungen, die schon oben angenommen wurden.

Da die Laser-Doppler Geräte einen Wert berechnen, der einer relativen Durchblutung Blood-Flow entspricht, kann in einer Näherungslösung für relative Werte auch folgende Gleichung herangezogen werden.

$$O_{2_{Umsatzrate}} = (SO_{2_{arteriell}} - SO_{2_{venös}}) \cdot H \cdot BloodFlow$$

Es folgt eine Darstellung der Berechnung der Primärinformationen:

Für die Berechnung der Sättigungswerte des Hämoglobins $SO_2$ kann z. B. die spektrale Form der Spektren über ein Formerkennungs- und Mischverfahren ausgewertet werden. Als Meßwerte erhält man die Sättigung des Hämoglobins mit $O_2$ im kapillär-venösen Gewebebett (beschrieben bei W. Dümmler, Diss. Uni-Erlangen, 1998).

In der vorliegenden speziellen Fragestellung der Bestimmung von Hb-Spektren durch Gewebemessungen wird die Absorption A dargestellt als Summe, gebildet aus Grundabsorption $A_o$ und den Mischabsorptionsanteilen von 0 % und 100 % oxygeniertem Hämoglobin. Die spektralen Absorptionskoeffizienten werden durch die spezifischen Extinktionswerte von oxygeniertem Hb, $\varepsilon^{Hb}{}_{ax}$ und deoxygenierten Extinktionskoeffizienten von Hb, $\varepsilon^{Hb}{}_{deax}$ in folgender Gleichung ausgedrückt.

$$A(\lambda) = A_o + C_{ox} \cdot \varepsilon^{Hb}_{ox}(\lambda) + C_{deox} \cdot \varepsilon^{Hb}_{deox}(\lambda)$$

Die Koeffizienten $C_{ox}$ und $C_{deox}$ geben den Mischungsanteil an, aus dem sich jedes gemessene Spektrum entsprechend seinem Oxygenierungsgrad zusammensetzen läßt.

Die Streuung S wird in einem ersten Ansatz als wellenlängenabhängige Funktion erster Ordnung angenähert, bestehend aus der Linearkombination von Grundstreuung $S_0$ und wellenlängenabhängigem Streuanteil $S_1$.

$$S(\lambda) = S_o + \lambda \cdot S_1$$

Nach dem oben beschriebenen Verfahren werden die gemessenen Spektren auf die Form A/S gebracht und mit dem Modellansatz, siehe rechte Seite der folgenden Gleichung, gleichgesetzt.

$$\frac{A}{S} = \frac{A + C_{ox} \cdot \varepsilon_{ox}^{Hb}(\lambda) + C_{deox} \cdot \varepsilon_{deox}^{Hb}(\lambda)}{S_0 + \lambda \cdot S_1}$$

Durch iterative Bestimmung der Koeffizienten $\frac{A_o}{S_o}, \frac{C_{ox}}{S_o}, \frac{C_{deox}}{S_o}, \frac{S_1}{S_o}$ mittels Newton und Least Square Verfahren und anschließender Quotientenbildung wird die Sättigung des Hämoglobins bestimmt:

$$SO_2 = \frac{C_{ox}}{C_{ox} + C_{deox}}$$

Die Sättigung des Hämoglobins kann somit nur im Wertebereich von 0 % bis 100 % liegen. Die Berechnungsgenauigkeit hängt von der Güte des Gewebemodells ab. Das oben präsentierte Gewebemodell ist jederzeit erweiterbar, so kann die Grundabsorption $A_o$ ersetzt werden durch gewebespezifische Grundspektren $A_{Gewebe}(\lambda)$, die aus einer Organtabelle zu entnehmen sind.

Zu Beginn jeder Messung sollte zur Verbesserung der Spektrometerwerte ein Dunkelspektrum aufgenommen werden, um die elektronische Null der Verstärker und den Wert des Störlichtes, das in die Detektoreinheit einfällt, zu erfassen. Zweitens ist es notwendig, ein Spektrum über einen Weißstandard aufzunehmen, um die Gerätefunktion der Lampe, des Sensors und der gesamten Detektoreinheit erfassen zu können. Je nach Güte des Spektrometers sollte in bestimmten Zeitintervallen die spektrale Genauigkeit durch eine spektrale Kontrollmessung z. B mittels einer Quecksilber-Argon Kalibrierquelle durchgeführt werden. Die beschriebenen Abgleichspektren sollten bevorzugt mit mindestens 10 mal höherer Mittelrate aufgenommen werden als die nachfolgenden Gewebespektren, da die Fehler im Dunkel- und im Weißstandardspektrum durch die spektrale Vorverarbeitung auf alle Meßdaten weitergereicht werden.

Die aufgenommen Rohspektren müssen vorverarbeitet werden, bevor sie zur Auswertung herangezogen werden können. Das Rückstreuspektrum $R(\lambda)$ setzt sich zusammen aus:

$$R(\lambda) = \frac{Spektrum_{Roh}(\lambda) - Spektrum_{Dunkel}(\lambda)}{Spektrum_{Weißstandard}(\lambda) - Spektrum_{Dunkel}(\lambda)}$$

Dazu muß das Spektrometer eine Calibrierungsroutine durchlaufen, während der das Dunkelspektrum und das Weißstandardspektrum aufgenommen werden. Durch die Vorverarbeitung der Spektren werden die Farbfehler des optischen Systems des Gerätes entsprechend dem Stand der Technik eliminiert.

Für die Berechnung der Hämoglobinoxygenierung im VIS und NIR Bereich werden vollständig oxygenierte Hämoglobinspektren $\varepsilon^{Hb}{}_{ax}(\lambda)$ und vollständig deoxygenierte Hämoglobinspektren $\varepsilon^{Hb}{}_{deax}(\lambda)$ benötigt. Diese Spektren sollten mit derselben Wellenlängenauflösung aufgenommen werden, in der auch die Meßspektren digitalisiert werden.

Das von W. Dümmler (Diss. Uni-Erlangen, 1998) dargestellte Gewebemodell kann erfindungsgemäß erweitert werden, so daß die spezifischen organspektren $A_{Gewebe}(\lambda)$ direkt in das Modell mit einfließen.

$$\frac{A}{S}(\lambda) = \frac{A_{Gewebe}(\lambda) + C_{ox} \cdot \varepsilon_{ox}^{Hb}(\lambda) + C_{deox} \cdot \varepsilon_{deox}^{Hb}(\lambda)}{S_0 + \lambda \cdot S_1}$$

Die spezifischen Gewebespektren sind für jedes Organ als ein typisches Mittelwertsspektrum unter hämoglobinfreier Perfusion zu gewinnen.

Aus vielen Messungen stammt die Erkenntnis, daß rückgestreute Hämoglobinspektren von Erythrozyten verzerrt und insbesondere gestaucht sind gegenüber Spektren, die in Transmissionsanordnung von Lichtquelle und Detektor aufgenommen wurden. Die Eigenschaften der einzelnen Spektrometerbausteine spielen dabei keine Rolle. Die Amplituden der Hämoglobinspektren, die in Remissionsanordnung gemessen werden, sollten den Amplituden der Hb/$HbO_2$-Referenzspektren ähnlich sein. Hieraus ergibt sich erneut die Forderung nach jeweils geeigneten, vergleichbaren Hb/Hb02-Referenzspektren. Der Hauptgrund für die Stauchung der Spektren liegt, nach heutigem Erkenntnisstand, in den unterschiedlichen Meßvolumina der verschiedenen Absorptionswerte des Hämoglobins. Das Licht der Wellenlängen zwischen 540 nm und 580 nm erfährt eine stärkere Abschwächung als in den angrenzenden Wellenlängenbereichen und dringt deshalb weniger tief in das Gewebe ein. Licht der schwächer absorbierenden Wellenlängen des Hämoglobin-Spektrums hingegen dringt tiefer in das Gewebe ein und erfährt deshalb, absolut gesehen, einen höheren Abschwächungsgrad, als unmittelbar vom Extinktionswert zu schließen wäre. Dieser Zusammenhang konnte direkt aus den Messungen der Eindringtiefen in den Zweischichtmodellen gefolgert werden (siehe A. Krug, Diss. Uni-Erlangen, 1998).

In Fig. 8 wird ein Zweischichtmodell beschrieben, das aufgebaut ist aus einer streuenden Suspension (z. B. Intralipid ®, Zellen oder Gewebeschichtungen), angeordnet über einem totalen Absorber, wie z. B. Tinte, getrennt durch eine nur wenige µm dicke PVC-Folie. Zwei Lichtleiter reichen in die Suspension, der erste leitet Licht ein, der zweite nimmt rückgestreutes Licht auf. Die Anordnung dient zur Bestimmung der 90 % Eindringtiefe, die auch als Detektionstiefe bezeichnet wird.

Beispielhafte Ergebnisse aus Messungen der Detektionstiefen in Intralipid-Suspension sind in der Tabelle 2 zusammengestellt. Die Gegenüberstellung zeigt, daß bei 542 nm weitaus geringere Detektionstiefen bestimmt werden als bei 628 und 760 nm, wenn Hämoglobin das Meßvolumen aufgrund seiner unterschiedlichen Absorptionskoeffizienten für die verschiedenen Wellenlängen moduliert.

Tabelle 2: Berechnung der Detektionstiefen, aufgenommen mit 200 µm Quarzfasern in Intralipid-Suspension.

| Detektionstiefen in µm für Tinte als Hintergrundabsorber | | | |
|---|---|---|---|
| Wellenlänge | 542nm | 628nm | 760nm |
| IL 2% | 920 | 1000 | 1120 |
| IL2%+0,25 g Hb/dl | 320 | 580 | 880 |
| Detektionstiefen in µm für schwarzes PVC als Hintergrundabsorber | | | |
| Wellenlänge | 542nm | 628nm | 760nm |
| IL 2% | 1000 | 1040 | 1060 |
| IL2%+0,25 g Hb/dl | 380 | 660 | 920 |

Prinzipiell sind zwei Wege beschreitbar, um das Problem der Verzerrung der rückgestreuten Hb-Spektren zu lösen. Entweder die Meßspektren werden entsprechend ihrer Hämoglobinkonzentration entzerrt, indem die Extinktionswerte auf das jeweilige effektive Meßvolumen bezogen werden, oder es wird eine ganze Schar von $HbO_2$-Referenzspektren mit unterschiedlichen Hämoglobinkonzentrationen und unterschiedlichen Verzerrungsgraden generiert und dem Auswertealgorithmus zur Verfügung gestellt. Der erst genannten Lösung ist der Vorzug zu geben, da hierbei Standard-Hämoglobinspektren aus Küvettenmessungen verwendet werden können, und die Bestimmung des Meßvolumens zudem für die quantitative Berechnung der Hämoglobinkonzentration verwendet werden kann.

Die Bestimmung der Hämoglobinmenge wird entsprechend der Literatur beschrieben durch nachfolgende Gleichung:

$$Hb_{Menge} = C_{Hb} \bullet V_{Meß}$$

Aus obiger Formel wird deutlich, daß zur Bestimmung der Hämoglobinmenge die beiden Größen Hämoglobinkonzentration $C_{Hb}$ (insbes. im VIS) und Meßvolumen $V_{Meß}$ meßtechnisch erfaßt und berechnet werden müssen.

Für die Bestimmung der Hämoglobinmenge im jeweiligen Meßvolumen können verschiedene theoretische Ansätze zur Anwendung kommen. Im einfachsten Fall wird die Hämoglobinkonzentration mit Hilfe des Gesetzes von Lambert-Beer (siehe nächste Formel) bestimmt:

$$Ext. = \log\frac{I_o}{I} = C_{Hb} \bullet \varepsilon_{Hb}(\lambda) \bullet d$$

Die Extinktion Ext. wird berechnet aus dem Logarithmus der ins Objekt eingestrahlten Lichtintensität $I_0$ im Verhältnis zu der aus dem Objekt austretenden Lichtintensität I. Entsprechend Lambert-Beer ist die Extinktion abhängig von der Hämoglobinkonzentration $C_{Hb}$, dem wellenlängenabhängigen Absorptionskoeffizienten $\varepsilon_{Hb}$ und der Küvettendicke d.

Die $Hb_{conc.}$ ist in gleicher Weise in oben beschrieben zu bestimmen. Die $Hb_{conc.}$ ist proportional zum Extinktionswert (oder auch der Optical-Density) bezogen auf das jeweilige Meßvolumen.

$$Hb_{conc.} = K_i \bullet \frac{OD.}{V_{Meß}} = K_i \bullet \frac{\log\frac{I_o'}{I}}{\varepsilon_{Hb} \cdot V_{Meß}}$$

Die Bestimmung des Meßvolumens wird weiter unten beschreiben.

Eine andere Möglichkeit zur Bestimmung der Hämoglobinkonzentration ist gegeben durch die Strahlungstransportgleichung. In ihrer allgemeinen Form ist sie jedoch nicht geschlossen lösbar und ist aus diesem Grund nicht besonders gut zu handhaben. Deshalb wird in der Gewebespektrometrie häufig mit der Diffusionsnäherung der Strahlungstransportgleichung gearbeitet.

Die Gleichungen, abgeleitet auf der Basis der Diffusionsnäherung, werden nachfolgend eingeführt. Mit Hilfe der Diffusionsapproximation wird ein x-Gradient in Remissionsanordnung der Lichtleiter formal beschrieben als:

$$I(x) = \frac{3P_o}{16\pi^2}\frac{\exp\left(-x\sqrt{3\mu_a\left(\mu_a + \mu_s'\right)}\right)}{x}\left(\mu_a + \mu_s'\right).$$

Hieraus läßt sich mit einer Zusammenfassung zu zwei Koeffizienten $C_1$ und $C_2$

$$\begin{aligned} C_1 &= \frac{3P_o}{16\pi^2}\left(\mu_a + \mu_s'\right) \\ &= \frac{3P_o}{16\pi^2}\left(\rho\sigma_a + \rho\sigma_s(1-g)\right) \end{aligned}$$

und

$$C_2 = \sqrt{3\mu_a\left(\mu_a + \mu_s{}'\right)} = \sqrt{3\rho\sigma_a\left(\rho\sigma_a + \rho\sigma_s(1-g)\right)} = \rho\cdot\sqrt{3\sigma_a\left(\sigma_a + \sigma_s(1-g)\right)}$$

die Beschreibung der Gradienten zusammenfassen zu:

$$I(x) = C_1 \frac{\exp\left(-C_2 x\right)}{x}$$

Aus den ermittelten Koeffizienten $C_1$ und $C_2$ kann nachfolgend $\mu_a$ und $\mu_s$ bestimmt werden. Der Koeffizient $\mu_a(\lambda)$ gibt den Absorptionskoeffizienten des Gewebes wieder, aus dem mit entsprechenden Näherungen oder nach Vernachlässigung weiterer Absorber im Gewebe die Hämoglobinkonzentration im Gewebe bestimmt werden kann.

Die quantitative Bestimmung der lokalen Hämoglobinkonzentration im Mikrovolumen durch Auswertung der Rückstreuspektren ist eine komplexe Aufgabe. Von den verschiedenen Arbeitsgruppen wurden die optischen Eigenschaften unterschiedlicher Gewebe untersucht. Es zeigte sich, daß der Streukoeffizient $\mu_s$ mindestens 10 mal größer ist als der Absorptionskoeffizient $\mu_a$. Demzufolge wird die rückgestreute Lichtmenge primär bestimmt durch die Streueigenschaften des untersuchten Gewebes.

Fig. 9 zeigt drei Spektren, nämlich das Spektrum der Gerätefunktion $I_{Gerät}(\lambda_i)$, das Spektrum eines idealen Streuers $I'_o(\lambda_i)$ und ein gemessenes Hämoglobinspektrum $I_m(\lambda_i)$.

Für die Entwicklung des nachfolgenden Bestimmungsverfahrens zur Berechnung der Hämoglobinkonzentration sind die beiden nachfolgenden Aussagen wichtig:

1. An der Gewebeoberfläche können nur Lichtintensitäten gemessen werden, die durch Streuung im Gewebe zurückgeworfen wurden.
2. Befindet sich ein Absorber, wie das Hämoglobin, im Gewebe, so schwächt dieser das Licht auf dem Weg ins Gewebe zwischen den Streuereignissen und auf dem Weg zurück zum Detektorlichtleiter.

Aus beiden Aussagen läßt sich folgern, daß bei einer Wellenlänge, bei der die Absorption vernachlässigbar klein ist, die gemessene Lichtintensität nur von der Rückstreuung bestimmt ist. Bei allen anderen Wellenlängen, bei denen die Absorption nicht vernachlässigbar ist, wird das Licht durch den Absorber geschwächt und die Intensität ist deshalb kleiner als die ungestörte Rückstreuintensität.

So wurde erfindungsgemäß ein neues Auswerteverfahren zur Bestimmung der Hb-Amplituden von Gewebespektren entwickelt. Der erste Teil wurde schon in der Dissertation von A. Krug (1998, Uni-Erlangen) veröffentlicht. Neu ist das Verfahren, bei dem die Zwischenergebnisse der Hb-Bestimmung durch Extraktion von Hämoglobinamplituden aus den Rückstreuspektren auf den durch ein zweites Verfahren beschriebenen Meßvolumenwert an der gleichen Meßstelle bezogen werden. Hieraus entstehen erfindungsgemäß ständig auf das aktuelle Meßvolumen bezogene Hämoglobinkonzentrationswerte.

Der Zwischenwert der relativen Hämoglobinkonzentration kann auch aus Lösungen der Diffusionsgleichung ermittelt werden.

In Fig. 9 sind unkorrigierte Spektren dargestellt. Die Kurve $I_{Gerät}(\lambda_i)$ zeigt die Gerätefunktion, bzw. die optische Fehlerfunktion des Gewebespektrometers. Gegen dieses Spektrum müssen alle gemessenen Spektren korrigiert werden, um die gerätespezifischen Verfälschungen der Meßspektren zu eliminieren. Die Kurve $I'o(\lambda_i)$ entspricht dem Spektrum, das man erhält, wenn ein weißer Streuer vorliegt. Die Kurve $I_m(\lambda_i)$ entspricht einem tatsächlich gemessenen Spektrum, das man bei einer physiologischen Hämoglobinkonzentration im Streumedium erhält.

Kennt man die reine Rückstreuintensität $I'_o(\lambda_i)$, so kann man den Anteil der Lichtschwächung durch den Absorber aus der Differenz zwischen der reinen Rückstreuintensität und der gemessenen Intensität $I_m(\lambda_i)$ ermitteln. Der formale Zusammenhang läßt sich ausdrücken durch

$$I'_o(\lambda_i) = I_m(\lambda_i) + \Delta I_{Abs}(\lambda_i)$$

In der Rückstreuphotometrie erhalten wir die ungeschwächte Rückstreuintensität $I'_o$ dann, wenn die Absorberkonzentration im Gewebe gleich Null ist. Wenn sich im Gewebe also ausschließlich Streuer befinden.

Die Fig. 10 zeigt ein Hämoglobinspektrum Im $(\lambda_i)$ mit Oxygenierungswerten von 0 bis 100 %, errechnet über das Farbmischverfahren zur Bestimmung der Flächenwerte der Hämoglobinämplituden. Die Annahme, daß eine Wellenlänge existiert, bei der die Absorption durch das Hämoglobin vernachlässigbar klein ist, existiert bei Wellenlängen größer 640 nm. Besser geeignet wären noch die Wellenlängen der absoluten Minima der Extinktionswerte des Hämoglobins, die bei 690 nm für das oxygenierte und bei 850 nm für das deoxygenierte Hämoglobin liegen.

Die Fig. 10 veranschaulicht, daß die Differenz zwischen $I'_o(\lambda_i)$ und $I_m(\lambda_i)$ der Absorption des Lichtes bei dieser Wellenlänge entspricht. Somit entspricht auch der Quotient

$$\log[I'_o(\lambda_i)\ /\ I_m(\lambda_i)]$$

der Absorption. Wird dieser Quotient ermittelt, so stellt er ein quantitatives Maß für die Extinktion bzw. für die Absorption des Lichtes in diesem ideal streuenden Medium dar.

Für die Berechnung der Hämoglobinkonzentration wird die Fläche der extrahierten Hämoglobinamplituden integriert und daraus ein Absorptionswert pro Spektrum berechnet. Die Integralwerte für oxygenierte und deoxygenierte Spektren unterscheiden sich jedoch. Deshalb wurde in der zweiten Entwicklungsstufe eine oxygenierungsabhängige Korrektur der extrahierten Hämoglobinamplituden oder der Flächen-Absorbtionswerte eingeführt. Die Grundidee dieser Korrektur besteht darin, die Flächeninhalte der unterschiedlich oxygenierten Spektren zu erfassen und eine Korrektur für die unterschiedlichen Oxygenierungswerte durchzuführen. Es wurde ermittelt, daß der Flächeninhalt eines voll oxygenierten Hb-Spektrums z. B. im Wellenlängenbereich von 500 - 630 nm um 16 % größer ist, als der Flächeninhalt eines voll deoxygenierten Spektrums (Siehe Fig. 10). Für die Ermittlung dieser Korrekturwerte wurde auf die Literaturspektren nach Assendelft, 1970, zurückgegriffen.

Durch Berechnungen mit einem Farbmischverfahren konnten auch die Flächeninhalte aller Zwischenwerte der Hb-Oxygenierung zwischen den Werten 0 % und 100 % $HbO_2$ ermittelt werden. Der Flächenwert der Gewebespektren wird ermittelt, nachdem diese in einem Normierungsschritt unabhängig vom Oxygenierungswert des Hämoglobins bestimmt werden.

Fig. 11 zeigt eine Anwendung des erfindungsgemäßen optischen Sauerstoffsensors zusammen mit einem Temperatursensor als Ohrsensor. Die Fig. 11 zeigt den Gehörgang mit Trommelfell, in den dieser spezielle Sensorkopf eingeführt wird. Aus Hygienegründen kann er mit einer transparenten Schutzfolie versehen sein. Diese Hygienekappe und der Sensor besitzen eine spezielle, dem Gehörgang angepasste Form und eine Art Anschlag, der eine Perforation des Trommelfells verhindert. Die Länge des Sensors bis zum Anschlag beträgt etwa 25 mm. Im Sensorkopf befindet sich ein optischer Sauerstoffsensor entsprechend den Fig. 1 bis Fig. 6 oder Fig. 21 oder auch Fig. 23. Entscheidend ist hierbei die Messung der Sauerstoffsättigung im Trommelfell, einer planaren Struktur. In der Kombination mit einem Temperatursensor erhält der Arzt die Informationen, die er benötigt.

Als Temperaturmeßverfahren kommen hier bevorzugt berührungslose Verfahren, wie die Infrarot-Temperaturmessung, oder die Verfahren der Temperaturmessung im Raum des abgeschlossenenen Gehörgangs oder des Innenohrs über NTC oder ähnliche Temperaturmessverfahren in Frage.

Da in dieser Applikationsform der optische Sauerstoffsensor nur in der als zweidimensionale Struktur betrachtbaren Haut des Trommelfells mißt, kann in diesem Spezialfall eine Reflexionsmessung anstelle einer Rückstreumessung, wie bei Messungen direkt auf Organoberflächen, durchgeführt werden. Aus diesem Grund ist die berührungslose Infrarot-Temperaturmessung gegenüber einem NTC-Sensor zu präferieren.

Für optische Messungen der Durchblutungsparameter auch im Mikrovolumen ist es wichtig, entsprechende Applikatoren zu entwickeln, die einerseits garantieren, daß der Sensor aufliegt und damit in direktem Kontakt zum Gewebe steht, sowie andererseits eine Bestimmung zulassen, wann der Anpreßdruck an das Gewebe zu hoch ist. Der Anpreßdruck darf einen bestimmten Wert nicht überschreiten, da sonst die oberflächennahe Perfusion der Kapillaren durch den Druck des Sensors beeinträchtigt wird und somit unter Umständen zu einer Fehlmessung führen kann.

Als Temperatursensoren kommen berührungslose Verfahren, wie die Infrarot-Temperaturmessung, oder Verfahren der Temperaturmessung im Raum des abgeschlossenen Außenohrs über NTC oder ähnlichen Temperaturmessverfahren in Frage.

In der nach Fig. 11 gezeigten Ausführungsform des Applikators sind nur Reflexionsmessungen möglich, da der Applikator nicht auf dem Trommelfell aufsitzt. Für Reflexionsmessungen genügen einkanalige Messungen mit dem Gewebespektrometer, mit dem pulsatilen Gewebespektrometer, mit dem Pulsoximeter, mit dem Laser-Doppler und/ oder der Temperatursonde. Für das Imageing von Gewebeparametern am Trommelfell, entsprechend Fig. 21 oder Fig. 23 muß der Ohrstöpsel verlängert werden und muß direkt auf das Trommelfell aufgesetzt werden. Für das 2D- und vor allem ein 3D-Imageing müssen wieder Rückstreumessungen anstelle von Reflexionsmessungen durchgeführt werden.

Fig. 12 zeigt eine Ausführung eines neuen Sensorkopfes. Dazu wird ein Sensorkopf entsprechend Fig. 1 bis 6, Fig. 21 oder Fig. 23 zusätzlich in eine Einheit integriert, die es ermöglicht, gleichzeitig zu detektieren, ob der Sensor sicher mit dem Gewebe in Kontakt steht, und ob der Anpreßdruck des Sensors nicht zu hoch ist, stellt damit also einen Druckindikator dar.

Diese Einheit kann als "Secure-Sensor-Applikator" bezeichnet werden. Sie enthält eine 1 bis 2 mm breite Nut, in der sich zwei Lichtleiterpaare an jeder Seite der Nut gegenüber stehen. Auf einer Seite zwei Sendefasern, auf der anderen Seite zwei Detektionsfasern. Das Sendelicht jeder Sendefaser wird bevorzugt jeweils mit einer anderen Frequenz amplitudenmoduliert. Diese Amplitudenmodulation macht den "Secure-Sensor-Applikator" gegenüber Fremdlichtquellen störsicher und erlaubt zudem eine Trennung der Lichtintensitäten, die aus den beiden Sendefasern kommen.

Wird der Sensor mit dem richtigen Druck appliziert, wird nur eine Lichtschwächung im Kanal 1 gemessen, während im Kanal 2 noch keine Abschwächung zu detektieren ist. Wird das Gewebe durch einen zu hohen Anpreßdruck zu stark deformiert, führt das zu einer verstärkten Ausbeulung der Haut oder der Gewebeoberfläche in die Nut des Sensors hinein, wodurch dann auch der Kanal 2 eine Abschwächung der Lichtübertragung erfährt. So signalisiert er einen zu hohen Applikationsdruck und dient zur Feststellung unzulässiger Meßbedingungen, die zu einer Minderperfusion der Kapillaren führen würde.

Fig. 13 zeigt die berechneten relativen Extinktionswerte (O.D.) bei 5 verschiedenen Hb-Konzentrationen.

Fig. 14 zeigt den funktionellen Zusammenhang zwischen der Hämoglobinkonzentration in einer Suspension und dem errechneten Mittelwert der relativen Extinktionswerte (O. D.).

Die beiden Fig. 13 und 14 fassen die Resultate eines Versuchs zusammen und dokumentieren die Gültigkeit des vorgestellten Verfahrens. Die Ergebnisse zeigen den Zusammenhang auf zwischen zupipetierten Hb-Konzentrationen in Suspension und den oxygenierungsabhängigen korrigierten Hb-Extinktionswerten. In Fig. 13 kann die Genauigkeit der oxygenierungsabhängigen Korrektur für alle Hämoglobinkonzentrationsstufen abgelesen werden, bei oxygenierungsänderungen von 0 % auf 100 % $HbO_2$. Die größte Abweichung kann bei 1,0 g/dl abgelesen werden.

Das Verfahren für die Berechnung der rel. Hämoglobinkonzentration $Hb_{conc}$ im NIR ist in ähnlicher Weise aufzubauen, wie dies für den sichtbaren Wellenlängenbereich entwickelt wurde. Es müssen jedoch die spektralen Besonderheiten im NIR berücksichtigt werden. Entscheidend für die Berechnungen mit dem Fitt-Algorithmus ist die Auswahl des betrachteten Wellenlängenbereichs, da der Fitt-Algorithmus nur bei entsprechend charakteristischem, spektralen Unterschied, abhängig von der Oxygenierung des Hämoglobins, anwendbar ist. Es zeigte sich, daß der Wellenlängenbereich von 600 - 900 nm besonders geeignet ist für Messungen im NIR-Wellenlängenbereich.

Fig. 15 zeigt die Abhängigkeit der Rückstreuintensität einerseits von der Sender-Detektor-Seperation (x-Gradient) und von der Eindringtiefe (z-Gradient). Der x-Gradient der Rückstreuintenitäten in Abhängigkeit von der Sender-Detektor-Separation und der z-Gradient können in einer Streuküvette nach Fig. 16 ohne Tinte bestimmt werden.

Die effektive Eindringtiefe, hier auch als Detektionstiefe bezeichnet, kann durch die in Fig. 16 gezeigte Einrichtung bestimmt werden (siehe A. Krug, Diss. Uni-Erlangen, 1998).

Fig. 16 zeigt eine Streuküvette gefüllt mit Streususpension und Tinte zur Definition mit Darstellung der Scan-Richtungen. Mit einer Mikrometerschraube wurde jeweils eine neue Lichtleiterseparation z eingestellt, die dann in x-Richtung gescannt wurde. Der Scan gibt die Lichtintensitäten der verschiedenen Abstände von den Lichtleitern zur Tinte - dem "schwarzen Loch" - wieder.

Fig. 17 zeigt ein Übersichtsbild von Berechnungen der Detektionstiefen mittels 90 % Schwelle in verschiedenen Intralipid-Suspensionen und bei verschiedenen Separationen, ausgewertet bei 760 nm.

Nun zur die Beschreibung der erfindungsgemäß neuen Berechnung des aktuellen Meßvolumens im VIS und im NIR: Das Meßvolumen $V_{meß}$ kann bestimmt werden aus der Messung eines oberflächlichen Intensitätsgradienten. Zusätzlich wird eine experimentell für das jeweilige Gewebe zu bestimmende Übertragungsfunktion benötigt.

Die Übertragungsfunktion, wie in Fig. 18 zu sehen, stellt den Zusammenhang her zwischen dem meßtechnisch erfaßbaren Intensitätsgradienten an der Oberfläche und dem nicht meßbaren Intensitätsgradienten in die Tiefe des Gewebes. Als effektives Meßvolumen wird das Volumen erachtet, in dem die Rückstreuintensitätsgradienten auf 90 % abgeschwächt wurden, wie Fig.15 zeigt. Die Übertragungsfunktion kann formal beschrieben werden durch:

$$I_{z\text{-Gradient}}(z) = \text{Übertragungsfunktion}^{-1} * I_{x\text{-Gradient}}(x)$$

Fig. 19 zeigt die Definition der Bestimmungsgrößen a, b und c einer Halbellipse zur Illustration des Meßvolumens, das von einem Lichtwellenleiter bei Illumination eines streuenden Gewebes gebildet wird.

Aus der Bestimmung der Detektionstiefen in x und in z-Richtung (siehe Fig. 15) läßt sich das Meßvolumen erfindungsgemäß näherungsweise bestimmen.

Bei halbelliptischer Annahme der Meßvolumnina ergibt sich das effektive Meßvolumen entsprechend Fig. 19 zu:

$$V_{Meß\text{-}eff} = \frac{2}{3}\pi\, a\, b\, c$$

Die Bestimmungsgrößen der Ellipse a, b und c lassen sich durch die Berechnung der effektiven Eindringtiefen aus den Intensitätsgradienten ermitteln. Aufgrund der Rotationssymmetrie der Illumination kann

$$a = b = x_{eff},$$

der effektiven Eindringtiefe $x_{eff}$ in lateraler Richtung gesetzt werden. Die Tiefe c läßt sich aus der effektiven Eindringtiefe in transversaler Richtung bestimmen und es wird

$$c = z_{eff}$$

über die effektive Eindringtiefe in Z-Richtung, die senkrecht in das Gewebe gerichtet sein soll, bestimmt. Damit sind die 3 Parameter des halb-ellipischen Volumenintegrals und damit des relevanten Meßvolumens bestimmt.

In der Arbeit von A. Krug [Krug, Dissertation Uni Erlangen-Nürnberg, 1998] wurde vorgestellt, daß das Meßvolumen bei ansteigender Absorberkonzentration verringert wird. Korrigiert man die gemessenen Rückstreuextinktionswerte gegen das jeweils verringerte Meßvolumen, so läßt sich zeigen, daß man auch bei Messungen in stark streuenden Medien einen linearen Zusammenhang zwischen Absorbermenge in der Streususpension und den auf das aktuelle Meßvolumen bezogenen Extinktionswert erhält.

Erfindungsgemäß wird ein Verfahren zur Bestimmung der arteriellen Sauerstoffsättigung mittels breitbandigem Gewebespektrometer unter Einbeziehen der gesamten spektralen Information vorgeschlagen.

In einem ersten Ansatz wird der arterielle Sättigungswert des Hämoglobins bestimmt über das übliche Pulsoximeterverfahren mit der Auswertung von mindestens zwei Wellenlängen, wobei ein pulssynchrones Differenzsignal des Herzschlages gebildet wird. Vorzugsweise sollten diese Wellenlängen so ausgewählt werden, daß auch diese arteriellen Werte tiefenselektiv bestimmt werden können, und so, daß bei diesen Wellenlängen additiv die Leistung der monochromatischen als auch der Breitbandlichtquelle zum tragen kommen. In einem ersten Ansatz wird das übliche Pulsoximeterverfahren in den integrierten Sensorkopf mit einbezogen.

Erfindungsgemäß ein neuer Ansatz ist, die arterielle Sättigung über die breitbandigen Rückstreuspektren des Gewebephotometers zu bestimmen. Das Gewebephotometer erlaubt über das oben beschriebene Verfahren zur Bestimmung der Sättigung des Hämoglobins die Berechnung der aktuellen Sättigung des Hämoglobins im Meßvolumen. Über ein besonders schnelles Photometer mit Abtastzeiten im Bereich von 1-10 ms pro Wert ist es möglich, die pulssynchronen Änderungen der Sättigung zu erfassen. Das Gewebephotometer erfaßt immer einen Mittelwert, entsprechend dem Volumenmischungsverhältnis von arteriellen und kapillar-venösen Sättigungswerten.

Erfindungsgemäß wird der arterielle Puls durch Signalauswertung im Laser-Doppler erfaßt und dient zur Triggerung des Gewebespektrometers.

In Folge des systolischen Blutdruckes erhält man im Gewebe einen Anstieg des Blutflusses und des Blutvolumens. Entsprechend der Theorie der Pulsoximetrie wird zu diesem systolischen Blutvolumenanstieg "frisches", vollständig gesättigtes, arterielles Blut in das Gewebe geschoben. Hieraus resultiert auch in der Summe eine höhere Sättigung des Blutes im Meßvolumen des Gewebephotometers. Bestimmt man nun zur systolischen Blutvolumenaddition, das diastolische Blultvolumen und die Sättigung während der Systole und Diastole, so lassen sich mit der umgestellten Mischungsgleichung die arteriellen Sättigungen bestimmen.

$$SO_{2\,Misch.\,Syst.} \cdot Hb_{Menge_{Misch.\,Syst.}} = SO_{2\,art.} \cdot \Delta Hb_{Menge_{art.}} + SO_{2\,Diast.} \cdot Hb_{Menge_{Diast.}}$$

$$SO_{2\,art} = \frac{SO_{2\,Syst.} \cdot Hb_{Menge\,Syst.} - SO_{2\,Diast.} \cdot Hb_{Menge\,Diast.}}{\Delta Hb_{Menge\,art.}}$$

In Fig.20 sind zwei Beispielspektren gezeigt. Das zu 80 % gesättigte Spektrum entspricht einem Zustand am Ende der Systole, wenn der Gehalt an frischem sauerstoffreichen Blut größer ist als zur Zeit des Intervalls der diastolischen, langsameren Durchblutung, während der die Sättigung im wesentlichen der kapillar-venösen Sättigung entspricht. Das Blutvolumen $\Delta Hb_{Menge,\,art.}$ wird gebildet aus der Differenz der Blut- bzw. Hämoglobinmenge zum end-systolischen und end-diastolischen Zeitpunkt. Auch hier kommt das Verfahren zur Hämoglobinbestimmung, wie oben beschrieben, zur Anwendung.

Die Sättigungen $SO_{2\,syst.}$ und $SO_{2\,diast.}$ werden, ebenso wie oben beschrieben, mit der Kurvenauswertung der Gewebespektren bestimmt.

Das Pulsoximeterverfahren sollte jedoch auch aus dem spektrometrischen Datensatz als pulssynchrones Differenzsignal bestimmt werden, um durch die stark verbreiterte spektrale Datenbasis validere Aussagen ziehen zu können. Besonders interessant ist die Auswertung des Differenzsignals der spektrometrischen Daten, da dieses Differenzsignal bezogen werden kann auf den kapillär-venösen Basiswert und somit erstmals ein quantitativer arterieller Sauerstoffsättigungswert bestimmbar wird (siehe Fig.20).

Fig. 20 zeigt zwei Absorptionsspektren und deren Differenzsignal während der pulssynchronen Änderung der Spektren unter der Annahme konstanter Hämoglobinkonzentrationen im Meßvolumen.

In einer weiteren Ausführung der Erfindung wird ein zweidimensionales und ein dreidimensionales Imaging der lokalen Sauerstoffparameter (entsprechend Tabelle 1) über ein bildgebendes Verfahren vorgestellt:

Die bisherige Beschreibung der Erfindung bezog sich auf punktuelle Messungen im Umfeld der Beleuchtungsquelle, die entweder aus einer Laserquelle, aus LED's oder aus einer Weißlichtquelle besteht.

In der Medizin jedoch sind viele bildgebende Verfahren gefragt, begonnen mit Röntgenaufnahmen, Ultraschallbildern, bis hin zu kernspintomographischen Aufnahmen, die dem geschulten Auge des Arztes sehr leicht zugänglich sind. Informationen, die in Form von Bildern zusammengefaßt sind, bieten zudem eine ausgezeichnete Möglichkeit, eine große Fülle von Information mit hohem Ordnungsgrad zu vermitteln. Es wird hier ein Verfahren zur Aufnahme von zunächst zweidimensionalen und nachfolgend von dreidimensionalen Bildern der lokalen Verteilung der Sauerstoffparameter, die mit dem oben beschrieben Sensor meßbar sind, vorgestellt.

Die Sensortechnik zur Aufnahme der verschiedenen lokalen Sauerstoffparameter, wie in Tabelle 1 aufgelistet, wurde in den vorangegangen Abschnitten erklärt und soll hier als Grundlage dienen. Im Unterschied zur oben dargestellten Sensortechnik mit punktueller Erfassung der Sauerstoffparameter wird nachfolgend ein neues, primär zweidimensionales Imageingverfahren, erläutert.

Die Fig. 21 zeigt den prinzipiellen Aufbau der erfindungsgemäßen Aufnahmevorrichtung für zweidimensionale Abbildung von Sauerstoffparametern. In dieser Variante besteht das Kernstück des Sensors aus einem bildgebenden Lichtleiter (auch Bildbündel genannt), wie er bereits bei Endoskopen oder bei Kathetern Anwendung gefunden habt, um die Bildinformation aus dem Inneren des Körpers nach außen zu leiten. Der bildgebende Lichtleiter ist aus einem Bündel von Einzelfasern aufgebaut, die jedoch so geordnet sind, daß die Bildinformation erhalten bleibt. Dieser bildgebende Lichtleiter wird zwischen dem Gerät und der Meßstelle am Objekt angeordnet. Im Meßgerät wird nun dieser bildgebende Lichtleiter abgerastert mit der wie oben beschrieben direkt am Objekt plazierten punktuellen Meßsonde. Der immense Vorteil dieser Anordnung liegt in der Tatsache, daß alle bewegten Teile im Meßgerät angebracht werden können, und der Sensor, in diesem Falle das Ende des bildgebenden Lichtleiters, direkt an der zu messenden Oberfläche fixiert werden kann. Der neue, bildgebende Sensor braucht nur einmal fixiert zu werden. Durch das Scannen im Gerät wird das Objekt nicht durch die Scannerbewegung selbst verschoben, es braucht nur noch die plane geräteseitige Oberfläche des bildgebenden Lichteiters abgerastert werden. Diese Oberfläche kann mit größerer Geschwindigkeit abgerastert werden, da hierfür die mechanischen Bedingungen im Gerät besser zu bestimmen sind.

Entscheidend ist, daß die Einzelfaserdurchmesser im bildgebenden Lichtleiter kleiner gleich den Querschnitten der Punktmeßsonde sind. Hierdurch ist immer gewährleistet, daß über den bildgebenden Lichtleiter dieselbe Sensorgeometrie erzeugt wird, wie vorher auch direkt durch den Punktsensor definiert. Der Punktsensor wird um so exakter abgebildet, um so dichter die Packung der Fasern ist, und um so dünner die Einzelfasern des bildgebenden Lichtleiters sind. Die zweite Alternative, die exakte 1:1 Koppelung der Einzelfasern des Punktsensors in jeweils exakt eine Einzelfaser des bildgebenden Lichtleiters ist auch möglich, jedoch weitaus aufwendiger.

Es können erfindungsgemäß auch zeitgleich mehrere Punktsensoren verwendet werden. Das erfordert dann eine mehrkanalige Illuminationsquelle und eine mehrkanalige Detektionseinheit. Hierdurch ist es möglich, die Zeit für ein komplettes Image zu verringern, da dann bei Anordnung der Punktsensoren nebeneinander, zum Beispiel in y-Richtung, nur noch die halbe Anzahl von Scans in x-Richtung durchgeführt werden müßten.

Alternativ zum bildgebenden Lichtleiter, oder als Ersatz für den Scanner und der gesamten Detektionseinheit, können auch andere Kameras, wie CCD-Kameras mit einer Zusatzeinheit, verwendet werden, die direkt eine spektrale Zerlegung der detektierten Information ermöglichen. Bisher erlauben diese Zusätze auf den Kameras noch keine ausreichende spektrale Auflösung, doch mit einer weiterentwickelten Technik könnte dies bald realisiert werden.

In Fig. 22 wird das Bild einer abgerasterten durchbluteten Leberoberfläche gezeigt, das die lokale Verteilung der Sättigung des Hämoglobins darstellt. In diesem noch sehr aufwendigen Verfahren wurde der Punktsensor noch direkt auf der Leberoberfläche verschoben. Die Entkoppelung der Bewegung des Punktsensors von der Organoberfläche wurde durch eine dazwischen gespannte PVC-Folie erreicht. Das Image bildet die Verteilung der Sättigung des Hämoglobins einer isoliert perfundierten Leberoberfläche ab. Entsprechend der Zusammenstellung von optischen Verfahren entsprechend Tabelle 1 lassen sich auch Images der anderen genannten Sauerstoffparameter und der anderen Gewebefarbstoffe entsprechend Tabelle 3 gewinnen.

Es zeigte sich bei diesen Versuchen, daß die Sensorgeometrie entscheidend ist für die räumliche Auflösung, mit der die Verteilung der Sauerstoffparameter abgebildet werden kann. Deshalb sollte entsprechend der morphologischen Struktur des Gewebes, das untersucht werden soll, eine entsprechende Sensorauflösung über die Faserdurchmesser, Apertur und Fasermaterial ausgewählt werden.

Ein dreidimensionales Aufnahmeverfahren zum Imaging von lokalen Sauerstoffparametern baut auf dem zweidimensionalen Aufnahmeverfahren auf. Es wird, wie Fig. 23 zeigt, eine ähnliche Art von bildgebendem Lichtleiter (auch Bildbündel genannt) benutzt, wie in Fig. 21 dargestellt ist. Im Gegensatz hierzu wird nun jedoch erfindungsgemäß der bildgebende Lichtleiter im Gerät mit dem tiefenselektiven Sensor aus Abbildung 1 mit einem x-y-Scanner abgerastert. Das zweidimensionale Imaging wird kombiniert mit der tiefenselektiven Aufnahme der Sauerstoffparameter über den Sensor mit mehreren Kanälen in unterschiedlicher Separation sowohl für die Dopplermessungen, als auch für die Detektion der rückgestreuten Intensitäten.

Eine Ausgestaltung der Erfindung erlaubt auch die Bestimmung weiterer Gewebeparameter mit den oben beschrieben spektrometrischen Meßverfahren:

Neben dem Chromophor Hämoglobin ist es interessant, weitere Farbstoffe (zusammengefaßt in Tabelle 3), die im Gewebe vorkommen, wie zum Beispiel Cytochrome, Myoglobin, Melanin und Bilirubin, spektrometrisch zu bestimmen.

Unter physiologischen Gewebebindungen sind die Messungen von weiteren Farbstoffen zeitgleich mit dem Hämoglobin sehr schwierig, da die Absorption, die durch das Hämoglobin hervorgerufen wird, die Spektren der anderen genannten Farbstoffe total überdeckt.
Physiologisch und klinisch interessant sind jedoch die Bestimmung der zusätzlich zum Hämoglobin genannten Gewebefarbstoffe. Bei hämoglobinfrei perfundierten Organen oder in pathologisch veränderter Situation ist die Bestimmung der genannten Gewebefarbstoffe möglich. Im hämoglobinfreien Zustand kann erfindungsgemäß der Redoxzustand der Cytochrome, zum Beispiel während der Transplantation von Organen, gemessen werden. Möglich ist auch die Untersuchung der Myoglobin-Sauerstoffsättigung und -Konzentration im Skelett- und Herzmuskel.

Die Verfahren zur Erstellung von 2-dimensionalen und 3-dimensionalen Images der Sauerstoffparameter entsprechend Tabelle 1 und/oder der abgeleiten Parameter entsprechend Tabelle 3, gewonnen aus Gewebewerten, durch die Kombination der Signale aus dem Gewebespektrometer und/oder dem Laser-Doppler und/oder dem Pulsoximeter und/oder Temperatursensor sind besonders in ihrer Kombination der Methoden neu.

Die Bedeutung eines Monitorings dieser Gewebesubstanzen liegt in der sich hiermit eröffnenden Möglichkeit, direkt intrazelluläre Sauerstoffversorgungsbedingungen untersuchen zu können. Der oben beschriebene Fitt-Algorithmus kann durch die Bereitstellung von vollständig reduzierten und vollständig oxidierten Cytochrom-Referenzspektren (siehe Fig. 24) auf die Berechnung des Redoxzustand der untersuchten Cytochrome umgestellt werden. Fig. 24 zeigt Cytochromspektren, oxidierte Cytochrome und reduzierte Cytochrome, gemessen in Mitochondrien-Suspension.

In gleicher Art und Weise kann, durch die Bereitstellung von vollständig oxygenierten und vollständig deoxygenierten Myoglobinspektren, auch die Sättigung des Myoglobins mit Sauerstoff über den oben beschriebenen Fitt-Algorithmus berechnet werden.

Das erfindungsgemäße spektrale Verfahren zur Bestimmung der Hämoglobinkonzentration aus Rückstreuspektren kann in ähnlicher Weise für die hier genannten Gewebefarbstoffe zur Bestimmung der intrazellulären Cytochrom-, Myoglobin-, Melanin- und Bilirubinkonzentrationen angewandt werden.

Zur Bestimmung der Cytochrom- und Myoglobin-Werte mit oben genannten Verfahren ist der Wellenlängenbereich von 500 - 650 nm besonders geeignet, da in diesem Wellenlängenbereich die von Natur aus niedrigen Absorptionskoeffizienten dieser zellulären Absorber die vergleichsweise höchsten Werte aufweisen. Deshalb sind in diesem Wellenlängenbereich die deutlichsten Absorptionsspektren mit dem besten Signal-zu-Rauschverhältnis zu erhalten.

Das Melanin, der Farbstoff der Haut, und das Bilirubin, ein Abbauprodukt des Hämoglobins, besitzen weniger charakteristische spektrale Kurvenformen als die Cytochrome. Das Myoglobin und das Hämoglobin und lassen sich deshalb nur weniger spezifisch und weniger eindeutig quantitativ erfassen.

Das Verfahren zur Bestimmung von Konzentrationen eines im Gewebe befindlichen Absorbers läßt sich selbstverständlich ausweiten auch auf künstlich injizierte Farbstoffe (Dyes). Somit können nicht invasiv Farb-Ein- und Auswaschkurven unmittelbar lokal im Gewebe ermittelt werden.

Tabelle 3: Farbstoffe und/oder Farbstoffkonzentrationen, die im Gewebe natürlicherweise vorkommen und/oder in das Gewebe eingebracht werden, die mit dem Gewebespektrometer mittels erfindungsgemäßer Verfahren bestimmt werden können.

| Farbstoff | Bestimmungsparameter | Verfahren |
|---|---|---|
| Hämoglobin | • Sauerstoffsättigung | Fitt-Algorithmus, mit Referenzspektren |
| | • Hämoglobinkonzentration | Flächenintegrationsver fahren mit Gewebenormierung |
| Cytochrome | • Redoxzustand | Fitt-Algorithmus, mit Referenzspektren. |
| | • intrazelluläre Cytochromkonzentration | Flächenintegrationsver fahren mit Gewebenormierung |
| Myoglobin | • Myoglobinoxygenierung | Fitt-Algorithmus, mit Referenzspektren |
| | • intrazelluläre Myoglobinkonzentration | Flächenintegrationsver fahren mit Gewebenormierung |
| Melanin | • Melaninkonzentration | Absorptionsbestimmung |
| Bilirubin | • Bilirubinkonzentration | Absorptionsbestimmung |
| künstliche Farbstoffe | • spezifische Farbstoffkonzentration | Absorptionsbestimmung |
| | • Permeabilitätstests | Farbauswaschverfahren |

Mit der erfindungsgemäßen Vorrichtung können also bevorzugt folgende Messungen und Berechnungen durchgeführt werden:

Für die Bestimmung des Sauerstoffgehaltes werden nur die Ausgangssignale des breitbandigen Gewebe-Rückstreuspektrometers ausgewertet. Aus den Gewebespektren wird über die Farbinformation die Sauerstoffsättigung ($SO_2$) ermittelt und aus der Lichtschwächung die Hämoglobinkonzentration ($Hb_{conc}$). Durch die mehrkanaligen, in verschiedenen Separationen, messenden Gewebespektrometersignalen können die Gewebewerte tiefenselektiv erfaßt und auf das zugehörige Meßvolumen, das online erfaßt wird, bezogen werden. Entscheidend ist eine mehrkanalige Spektrometermessung, denn diese Meßanordnung erlaubt den Bezug der $SO_2$-Werte und/oder der $Hb_{conc}$-Werte auf das Meßvolumen. Das Meßvolumen wird über Gradientenmessungen und nachfolgender Bestimmung von Absorption und Streuung bestimmt. Entscheidend sind die orts- und zeitgleichen Messungen über den gleichen Sensor (siehe Fig. 1), um den Bezug der Signale auf die jeweiligen Meßvolumina gewährleisten zu können.

Für die Bestimmung des Sauerstoffverbrauches in arteriell-venös gemischten Gewebe werden die Ausgangssignale des Gewebespektrometers zusammen ausgewertet mit den pulsatilen Signalen entweder eines Pulsoximeters oder eines schnellen Gewebespektrometers. Im Falle der pulsatilen Gewebespektrometermessung werden die größten Sättigungswerte während eines Herzzyklus differentiell ausgewertet, um nur den arteriellen Blutanteil diskriminieren zu können. Entscheidend ist die hohe Datenerfassungsrate, um den arteriellen Strompuls erfassen zu können. Ohne die Diskriminierung des Pulses kann keine arterielle $O_2$-Sättigung bestimmt werden. Die Triggerung des Meßzeitpunktes der arteriellen Bestimmung wird aus den Laser-Doppler-Signalen und/oder den zeitaufgelösten Signalen der $Hb_{conc.}$-Bestimmung definiert. Diese innovative Berechnungsverfahren zur Bestimmung der arteriellen Sättigung beruht auf der Auswertung der Signale eines schnellen breitbandigen Gewebespektrometers (<20 ms pro Taktlänge) und/oder der Triggerung durch das Signal der Laser-Doppler-Einheit. Entscheidend sind die orts- und zeitgleichen Messungen über den gleichen Sensor (siehe Fig. 1), um den Bezug der Signale auf die jeweiligen Meßvolumina gewährleisten zu können.

Die Gesamtblutmenge, auch als Gesamt-Gewebe-Hämoglobinmenge bezeichnet, wird bestimmt durch die Auswertung der mehrkanaligen, in verschiedenen Separationen zeitgleich durchgeführten, Gewebespektrometersignale. Aus den breitbandigen Gewebe-Rückstreuspektren wird die Hämoglobinkonzentration bezogen auf das Meßvolumen erfaßt. Durch Miteinbeziehen der Laborparameter Hämatokrit und Färbekoeffizient wird hieraus die Gesamtblutmenge im Meßvolumen ermittelt.

Die Sauerstofftransportkapazität wird ermittelt aus den Ausgangssignalen der mehrkanaligen Gewebespektrometermessung und dem Hämoglobinkonzentrationssignal, zusammen mit dem Blood-Flow-Ausgangssignal des Laser-Doppler Verfahrens. Entscheidend sind die orts- und zeitgleichen Messungen über den gleichen Sensor (siehe Fig. 1), um den Bezug der Signale auf die jeweiligen Meßvolumina gewährleisten zu können. Es ist wichtig, beide Verfahren gemeinsam einzusetzen, um die Sauerstofftransportkapazität bestimmen zu können. Der Blood-Flow alleine macht noch keine Aussage möglich über den Hämoglobingehalt der Erythrozyten und damit auch über die Sauerstoffbindungs- und Transportkapazität. Dia Hämoglobinkonzentration alleine wiederum gibt keine Auskunft über die Bewegung bzw. Geschwindigkeit der Erythrozyten.

Zur Bestimmung der lokal transportierten Sauerstoffmenge sind wieder mehrkanalige Ausgangssignale des Gewebespektrometers, das Sauerstoffsättigungssignal, die Hämoglobinkonzentration, die Ausgangssignale des Laser-Dopplers und die Blutflußgeschwindigkeit oder der Blood-Flow gemeinsam zu verrechnen, um größte Genauigkeit zu erzielen. Entscheidend für diese Signalgewinnung sind die orts- und zeitgleiche Erfassung der Gewebespekrometersignale und der Laser-Doppler Signale über dieselbe Sonde in damit vergleichbaren Meßvolumina. Entscheidend sind die orts- und zeitgleichen Messungen über den gleichen Sensor (siehe Fig. 1), um den Bezug der Signale auf die jeweiligen Meßvolumina gewährleisten zu können.

Die Sauerstoffverbrauchsrate in arteriell-venös gemischtem Gewebe ist eine relative Maßzahl, bei der auf einen absoluten Bezug der $Hbc_{onc}$. und der $SO_2$ auf das Meßvolumen verzichtet wird. Deshalb können hier auch einkanalige Spektrometer- und Pulsoximeter-Messungen durchgeführt werden. Es werden die Ausgangssignale des Gewebespektrometers ($SO_2$ und $Hb_{conc}$) und die Pulsoximetersignale oder die pulsatilen differentiellen Ausgangssignale des Gewebespektrometers (arterielles $SO_2$) zusammengeführt und zeitsynchron ausgewertet. Entscheidend sind die orts- und zeitgleichen Messungen über den gleichen Sensor (siehe Fig. 1), um den Bezug der Signale auf die jeweiligen Meßvolumina gewährleisten zu können.

Für die Bestimmung des Sauerstoffumsatzes werden die Primärsignale des breitbandigen Gewebespektrometers ($SO_2$ und $Hb_{Menge}$), des pulsatilen Gewebespektrometers, des pulsatilen Gewebespektrometers, des Pulsoximeters ($SO_{2\ art}$.) und/oder die Laser-Doppler- Primärsignale ($V_{Blut}$, $Menge_{Erys,bewegt}$) mehrkanalig ausgewertet, um den Meßvolumenbezug und/oder die Tiefenselektivität der Werte erzielen zu können. Der Sauerstoffumsatz gibt die Differenz des Volumenstromes von $O_2$, an, der arteriell antransportiert und venös abtransportiert wird. Entscheidend sind die orts- und zeitgleichen Messungen über den gleichen Sensor (siehe Fig. 1), um den Bezug der Signale auf die jeweiligen Meßvolumina gewährleisten zu können. Zudem ist die Meßvolumenerfassung durch spektrale Intensitätsgradienten entscheidend für den Bezug der Mengen-Maßzahl auf das gemeinsame Meßvolumen.

Für die Bestimmung der Sauerstoffumsatzrate in arteriell-venös gemischtem Gewebe wird auf den quantitativen Bezug auf das Meßvolumen verzichtet. Hierdurch sind auch ein-/oder mehrkanalige Gewebespekrometersignale ($SO_2$) und ein-/oder mehrkanalige pulsatile Gewebespekrometersignale und/oder Pulsoximetersignale ($SO_{2\ art.}$) sowie ein-/oder mehrkanalige Laser-Doppler-Signale (Blood-Flow) zur Errechung der Sauerstoffumsatzrate nötig.

Für die Bestimmung des lokalen Gewebesauerstoffpartialdrucks ($pO_2$) sind die orts- und zeitgleiche Erfassung der Primärsignale des Gewebespektrometers ($SO_2$) und der Temperatur (T), sowie der Laborparameter ($pCO_2$ und 2,3 BPG) nötig. Der lokale $pO_2$ kann näherungsweise bestimmt werden für kapillärvenöses und/oder arteriell versorgtes Gewebe, durch Diskriminierung von kapillär-venösem $SO_2$ und arteriellem $SO_2$ art.

Erfindungsgemäß ist auch ein Verfahren zur Bestimmung der lokalen Hämoglobinkonzentration aus Gewebespektren. Dieses ist normiert auf das approximierte Gewebegrundspektrum, entsprechend Fig. 9 mit gleichzeitigem Bezug der $Hb_{Menge}$, die aus den extrahierten Hb-Amplituden berechnet wird, auf das zugehörige Meßvolumen des Sensors. Die Bestimmung des Meßvolumens erfolgt aus Lichtintensitätsgradienten, die spektral ausgewertet werden, um hieraus die Übertragungsfunktion entsprechend Fig. 24 zu bestimmen und/oder die Absorptions- und Streukoeffizienten aller beteiligter Wellenlängen aus der Diffusionstheorie, auf die der $Hb_{Mengen}$ Wert bezogen wird.

Erfindungsgemäß sind auch Verfahren zur Bestimmung der arteriellen Sauerstoffsättigung durch differenzielle Auswertung der zeitaufgelösten spektralen Rückstreusignale entsprechend Fig. 20 mit Hilfe eines schnellen breitbandigen Gewebespektrometers. Möglich ist auch die Kombination mit einem Laser-Doppler-System zur Triggerung des arteriellen Strompulszeitpunktes für die Bestimmung des Sauerstoffsättigungswertes mit dem höchsten, also arteriellen Sättigungsanteils. Der Vorteil dieser Kombination liegt in der geringeren damit nur noch notwendigen Spektrenaufnahmerate des Gewebespektrometers.

## Patentansprüche

1. Vorrichtung zur Ermittlung des lokalen Sauerstoffumsatzes und/oder des Sauerstoffverbrauchs und/oder der $O_2$-Transportkapazität und/oder der transportierten $O_2$-Menge und/oder der Sauerstoffverbrauchsrate und/oder der Sauerstoffumsatzrate in durchblutetem Gewebe, ermittelt aus der lokalen Hämoglobinkonzentration und/oder der lokalen Sauerstoffsättigung und/oder der arteriellen Sauerstoffsättigung und/oder der Blutflussgeschwindigkeit und/oder der transportierten Blutmenge und/oder der Gewebetemperatur, mit einem optischen Sensor (S) zum Auflegen auf das Gewebe; durch den das Gewebe mit Licht einer Weißlichtquelle (W) und einer Laserlichtquelle (L) beleuchtbar ist, mit einem oder mehreren Detektoren (DD, DR)zum Empfangen des von dem Gewebe zurückgestreuten Lichtes und mit einer Auswerteeinheit, welche zur spektroskopischen und/oder spektrometrischen Auswertung des zurückgestreuten Weißlichtes und zur Laser-Doppler-Auswertung mittels dem zurückgestreuten Laserlicht ausgebildet ist.

**2.** Vorrichtung nach Anspruch 1, **dadurch gekennzeichnet, dass** der optische Sensor (S) die Weißlichtquelle (W), die Laserlichtquelle (L) und die Detektoren (DD, DR) aufweist.

**3.** Vorrichtung nach Anspruch 1, **dadurch gekennzeichnet, dass** die Weißlichtquelle (W) und die Laserlichtquelle (L) Licht über Lichtleitfasern zum Sensor (S) schicken und die Detektoren (DD, DR) vom Gewebe zurückgestreutes Licht über Lichtleitfasern empfangen.

**4.** Vorrichtung nach einem der vorhergehenden Ansprüche, **dadurch gekennzeichnet, dass** als Auswerteeinheit ein Laser-Doppler-Spektroskop und ein Spektrometer und/oder Spektroskop und/oder Gewebespektrometer und/oder Gewebespektroskop und/oder Pulsoximeter vorgesehen sind.

**5.** Vorrichtung nach Anspruch 4, **dadurch gekennzeichnet, dass** eine Temperatursonde (DT) und als Auswerteeinheit ein Temperaturmessgerät vorgesehen sind.

**6.** Vorrichtung nach Anspruch 3, **dadurch gekennzeichnet, dass** die Lichtleitfasern auf einer Kreisform um eine zentrale Faser oder eine Temperatursonde (DT) angeordnet sind.

**7.** Vorrichtung nach Anspruch 6, **dadurch gekennzeichnet, dass** je eine Faser für die Weißlichtquelle (W) und für den Laser (L), sowie jeweils mindestens zwei Detektionsfasern (DR, DD) auf einem Kreisbogen in definierten Abständen von den Beleuchtungsquellen liegen, die jeweils einer separaten Auswertung zugeführt werden.

**8.** Vorrichtung nach Anspruch 7, **dadurch gekennzeichnet, dass** die Detektionsfasern (DR) zusammen ausgewertet werden.

**9.** Vorrichtung nach Anspruch 3, **dadurch gekennzeichnet, dass** die beleuchteten Fasern für die Weißlichtquelle und/oder die Laserlichtquelle auf einem offenen oder geschlossenen Kreisbogen direkt um eine Zentralfaser liegen und über die Lichtquellen beleuchtet werden, wobei die Detektion der rückgestreuten und/oder Laser-Doppler Signale über die Zentralfaser erfolgt.

**10.** Vorrichtung nach Anspruch 9, **dadurch gekennzeichnet, dass** die beleuchteten Fasern (W) und/oder (L) auf einem größeren Radius und/oder auf verschiedenen Kreisradien liegen, die zeitgleich und/oder alternierend illuminiert werden.

**11.** Vorrichtung nach einem der Ansprüche 3 bis 10, **gekennzeichnet durch** ein Bündel an Lichtleitfasern, das vom Sensor (S) zum Detektor oder zu einer Kamera, wie einer Farb-CCD-Kamera, reicht, so dass eine zweidimensionale Abbildung der ausgewerteten Signale erzeugt werden kann.

**12.** Vorrichtung nach Anspruch 11, **gekennzeichnet durch** einen zusätzlich tiefenselektiven Sensor (S) oder eine tiefenselektive Auswertung, so dass eine dreidimensionale Abbildung der aufgenommenen Messwerte erzeugt werden kann.

**13.** Vorrichtung nach Anspruch 3, **dadurch gekennzeichnet, dass** die Fasern einer Separation $x_i$ gemeinsam illuminiert und/oder ausgewertet werden.

**14.** Vorrichtung nach einem der Ansprüche 3 bis 13, **dadurch gekennzeichnet, dass** über sich gegenüberstehende Lichtleiter und/oder Lichtaus- und Eintrittsbereiche ein Druckindikatorsignal generiert wird, das die Verformung des Gewebes und/oder einer Membran aufgrund der Sensorapplikation aufzeigt.

**15.** Vorrichtung zur Ermittlung des lokalen Sauerstoffumsatzes und/oder des Sauerstoffverbrauchs und/oder der $O_2$-Transportkapazität und/oder der transportierten $O_2$-Menge und/oder der Sauerstoffverbrauchsrate und/oder der Sauerstoffumsatzrate in durchblutetem Gewebe des Trommelfells, ermittelt aus der lokalen Hämoglobinkonzentration und/oder der lokalen Sauerstoffsättigung und/oder der arteriellen Sauerstoffsättigung und/oder der Blutflussgeschwindigkeit und/oder der transportierten Blutmenge und/oder der Gewebetemperatur, mit einem optischen Sensor (S), welcher in den Gehörgang des Ohres einführbar und im eingeführten Zustand zum Trommelfell beabstandet ist, wobei durch den optischen Sensor das Gewebe mit Licht einer Weißlichtquelle (W) und einer Laserlichtquelle (L) beleuchtbar ist, mit einem oder mehreren Detektoren (DD, DR) zum Empfangen des von dem Gewebe reflektierten Lichtes und mit einer Auswerteeinheit, welche zur spektroskopischen und/oder spektrometrischen Auswertung des reflektierten Weißlichtes und zur Laser-Doppler-Auswertung mittels dem reflektierten Laserlicht ausgebildet ist.

**16.** Verfahren zur Ermittlung des lokalen Sauerstoffumsatzes und/oder des Sauerstoffverbrauchs und/oder der $O_2$-Transportkapazität und/oder der transportierten $O_2$-Menge und/oder der Sauerstoffverbrauchsrate und/oder der Sauerstoffumsatzrate in durchblutetem Gewebe, ermittelt aus der lokalen Hämoglobinkonzentration und/oder der lokalen Sauerstoffsättigung und/oder der arteriellen Sauerstoffsättigung und/oder der Blutflussgeschwindigkeit und/oder der transportierten Blutmenge und/oder der Gewebetemperatur, wobei ein optischer Sensor (S) auf das Gewebe aufgelegt wird, das Gewebe durch den Sensor (S) mit Licht einer Weißlichtquelle (W) und einer Laserlichtquelle (L) beleuchtet wird, das von dem Gewebe zurückgestreute Licht detektiert wird, und die zurückgestreuten Anteile des Weißlichtes spektroskopisch und/oder spektrometrisch ausgewertet werden und mittels dem zurückgestreuten Anteil des Laserlichts eine Laser-Doppler-Auswertung durchgeführt wird.

**17.** Verfahren nach Anspruch 16, **dadurch gekennzeichnet, dass** zusätzlich die Temperatur des Gewebes gemessen und ausgewertet wird.

## Claims

**1.** Device for detecting local oxygen metabolism and/or oxygen depletion and/or $O_2$ transport capacity and/or the transported $O_2$ amount and/or the oxygen depletion rate and/or the oxygen metabolism rate in tissue flowed through by blood, ascertained from the local haemoglobin concentration and/or the local oxygen saturation and/or the arterial oxygen saturation and/or the blood flow speed and/or the transported blood amount and/or the tissue temperature, comprising an optical sensor (S) for placing on the tissue, by which the tissue can be illuminated by light of a white light source (W) and a laser light source (L), one or more detectors (DD, DR) for receiving the light backscattered by the tissue, and an evaluating unit which is constructed for spectroscopic and/or spectrometric evaluation of the backscattered white light and for laser-doppler evaluation by means of the backscattered laser light.

**2.** Device according to claim 1, **characterised in that** the optical sensor (S) comprises the white light source (W), the laser light source (L) and the detectors (DD, DR).

**3.** Device according to claim 1, **characterised in that** the white light source (W) and the laser light source (L) send light to the sensor (S) by way of optical fibres and the detectors (DD, DR) receive light, which is backscattered by the tissue, by way of optical fibres.

**4.** Device according to any one of the preceding claims, **characterised in that** a laser-doppler spectroscope and a spectrometer and/or spectroscope and/or tissue spectrometer and/or tissue spectroscope and/or pulsoximeter are provided as evaluating unit.

**5.** Device according to claim 4, **characterised in that** a temperature probe (DT) is provided and a temperature measuring instrument is provided as evaluating unit.

**6.** Device according to claim 3, **characterised in that** the optical fibres are arranged in a circular form around a central fibre or a temperature probe (DT).

**7.** Device according to claim 6, **characterised in that** a respective fibre for the white light source (W), and in each instance at least two detection fibres (DR, DD) for the laser (L), lie on an arc at defined spacings from the illumination sources and are respectively fed to a separate evaluation.

**8.** Device according to claim 7, **characterised in that** the detection fibres (DR) are evaluated together.

**9.** Device according to claim 3, **characterised in that** illuminated fibres for the white light source and/or the laser light source lie on an open or closed arc directly around a central fibre and are illuminated by way of the light sources, wherein the detection of the backscattered and/or laser-doppler signals is carried out by way of the central fibre.

10. Device according to claim 9, **characterised in that** the illuminated fibres (W and/or L) lie on a greater radius and/or on different circle radii, which are illuminated simultaneously and/or in alternation.

11. Device according to any one of claims 3 to 10, **characterised by** a bundle of optical fibres reaching from the sensor (S) to the detector or to a camera, such as a colour CCD camera, so that a two-dimensional imaging of the evaluated signals can be produced.

12. Device according to claim 11, **characterised by** an additional depth-selective sensor (S) or a depth-selective evaluation so that a three-dimensional imaging of the recorded measurement values can be produced.

13. Device according to claim 3, **characterised in that** the fibres of a separation $x_i$ are illuminated and/or evaluated in common.

14. Device according to any one of claims 3 to 13, **characterised in that** a pressure indication signal, which shows the deformation of the tissue and/or a membrane due to the sensor application, is generated by way of oppositely disposed optical conductors and/or light entry and exit regions.

15. Device for detecting local oxygen metabolism and/or oxygen depletion and/or $O_2$ transport capacity and/or the transported $O_2$ amount and/or the oxygen depletion rate and/or the oxygen metabolism rate in tissue, which is flowed through by blood of the eardrum, ascertained from the local haemoglobin concentration and/or the local oxygen saturation and/or the arterial oxygen saturation and/or the blood flow speed and/or the transported blood amount and/or the tissue temperature, comprising an optical sensor (S), which is introducible into the auditory canal of the ear and in the introduced state is spaced from the eardrum, wherein through the optical sensor the tissue can be illuminated by light of a white light source (W) and a laser light source (L), one or more detectors (DD, DR) for receiving the light reflected by the tissue, and an evaluating unit which is constructed for spectroscopic and/or spectrometric evaluation of the reflected white light and for laser-doppler evaluation by means of the reflected laser light.

16. Device for detecting local oxygen metabolism and/or oxygen depletion and/or $O_2$ transport capacity and/or the transported $O_2$ amount and/or the oxygen depletion rate and/or the oxygen metabolism rate in tissue flowed through by blood, ascertained from the local haemoglobin concentration and/or the local oxygen saturation and/or the arterial oxygen saturation and/or the blood flow speed and/or the transported blood amount and/or the tissue temperature, wherein an optical sensor (S) is placed on the tissue, the tissue is illuminated by the sensor (S) with light of a white light source (W) and a laser light source (L), the light backscattered from the tissue is detected and the backscattered components of the white light are evaluated spectroscopically and/or spectrometrically and a laser-doppler evaluation is carried out by means of the backscattered component of the laser light.

17. Method according to claim 16, **characterised in that** the temperature of the tissue is additionally measured and evaluated.

**Revendications**

1. Dispositif pour déterminer le métabolisme d'oxygène local et/ou la consommation d'oxygène et/ou la capacité de transport d'$O_2$ et/ou la quantité d'$O_2$ transportée et/ou le taux de consommation d'oxygène et/ou le taux de métabolisme d'oxygène dans un tissu irrigué, déterminés à partir de la concentration en hémoglobine locale et/ou de la saturation en oxygène locale et/ou de la saturation en oxygène artérielle et/ou de la vitesse de flux sanguin et/ou de la quantité de sang transportée et/ou de la température du tissu, comprenant un capteur optique (S) à poser sur le tissu, permettant d'éclairer le tissu avec la lumière d'une source de lumière blanche (W) et d'une source de lumière laser (L), comprenant un ou plusieurs détecteurs (DD, DR) pour recevoir la lumière renvoyée par le tissu et comprenant une unité d'analyse, qui est réalisée pour l'analyse spectroscopique et/ou spectrométrique de la lumière blanche renvoyée et pour l'analyse Doppler laser au moyen de la lumière laser renvoyée.

2. Dispositif selon la revendication 1, **caractérisé en ce que** le capteur optique (S) présente la source de lumière blanche (W), la source de lumière laser (L) et les détecteurs (DD, DR).

3. Dispositif selon la revendication 1, **caractérisé en ce que** la source de lumière blanche (W) et la source de lumière laser (L) envoient la lumière par l'intermédiaire de fibres optiques au capteur (S) et **en ce que** les détecteurs (DD, DR) reçoivent la lumière renvoyée par le tissu par l'intermédiaire de fibres optiques.

4. Dispositif selon l'une quelconque des revendications précédentes, **caractérisé en ce qu'**un spectroscope Doppler laser et un spectromètre et/ou un spectroscope et/ou un spectromètre tissulaire et/ou un spectroscope tissulaire et/ou un oxymètre de pouls sont prévus en tant qu'unité d'analyse.

5. Dispositif selon la revendication 4, **caractérisé en ce qu'**une sonde de température (DT) est prévue et **en ce qu'**un thermomètre est prévu en tant qu'unité d'analyse.

6. Dispositif selon la revendication 3, **caractérisé en ce que** les fibres optiques sont disposées en forme circulaire autour d'une fibre optique centrale ou d'une sonde de température (DT).

7. Dispositif selon la revendication 6, **caractérisé en ce que** respectivement une fibre optique pour la source de lumière blanche (W) et pour le laser (L), ainsi que respectivement au moins deux fibres optiques de détection (DR, DD) se trouvent sur un arc de cercle à des distances définies par rapport aux sources d'éclairage, les fibres optiques étant amenées respectivement à un dispositif d'analyse séparé.

8. Dispositif selon la revendication 7, **caractérisé en ce que** les fibres optiques de détection (DR) sont analysées ensemble.

9. Dispositif selon la revendication 3, **caractérisé en ce que** les fibres optiques éclairées pour la source de lumière blanche et/ou la source de lumière laser se trouvent sur un arc de cercle ouvert ou fermé directement autour d'une fibre optique centrale et sont éclairées par les sources de lumière, dans lequel la détection des signaux renvoyés et/ou des signaux Doppler laser s'effectue par l'intermédiaire de la fibre optique centrale.

10. Dispositif selon la revendication 9, **caractérisé en ce que** les fibres optiques éclairées (W) et/ou (L) se trouvent sur un plus grand rayon et/ou sur différents rayons de cercle qui sont illuminés en même temps et/ou en alternance.

11. Dispositif selon l'une quelconque des revendications 3 à 10, **caractérisé par** un faisceau de fibres optiques qui s'étend du capteur (S) au détecteur ou jusqu'à une caméra, comme une caméra CCD couleur, de sorte qu'une image bidimensionnelle des signaux analysés peut être générée.

12. Dispositif selon la revendication 11, **caractérisé par** un capteur (S) supplémentaire, sélectif en profondeur, ou un dispositif d'analyse sélective en profondeur, de sorte qu'une image tridimensionnelle des valeurs mesurées enregistrées peut être générée.

13. Dispositif selon la revendication 3, **caractérisé en ce que** les fibres optiques d'une séparation $x_i$ sont illuminées et/ou analysées ensemble.

14. Dispositif selon l'une quelconque des revendications 3 à 13, **caractérisé en ce qu'**un signal indicateur de pression est généré par des guides de lumière et/ou des zones de sortie et d'entrée de lumière opposé(e)s, le signal indiquant la déformation du tissu et/ou d'une membrane sur la base de l'application de capteur.

15. Dispositif pour déterminer le métabolisme d'oxygène local et/ou la consommation d'oxygène et/ou la capacité de transport d'$O_2$ et/ou la quantité d'$O_2$ transportée et/ou le taux de consommation d'oxygène et/ou le taux de métabolisme d'oxygène dans un tissu irrigué du tympan, déterminés à partir de la concentration en hémoglobine locale et/ou de la saturation en oxygène locale et/ou de la saturation en oxygène artérielle et/ou de la vitesse de flux sanguin et/ou de la quantité de sang transportée et/ou de la température du tissu, comprenant un capteur optique (S) apte à introduire dans le conduit auditif de l'oreille et qui, à l'état introduit, est espacé par rapport au tympan, dans lequel le capteur optique permet d'éclairer le tissu avec la lumière d'une source de lumière blanche (W) et d'une source de lumière laser (L), comprenant un ou plusieurs détecteurs (DD, DR) pour recevoir la lumière réfléchie par le tissu et comprenant une unité d'analyse qui est réalisée pour l'analyse spectroscopique et/ou spectrométrique de la lumière blanche renvoyée et pour l'analyse Doppler au laser au moyen de la lumière laser renvoyée.

16. Procédé pour déterminer le métabolisme d'oxygène local et/ou la consommation d'oxygène et/ou la capacité de transport d'$O_2$ et/ou la quantité d'$O_2$ transportée et/ou le taux de consommation d'oxygène et/ou le taux de métabolisme d'oxygène dans un tissu irrigué, déterminés à partir de la concentration en hémoglobine locale et/ou de la saturation en oxygène locale et/ou de la saturation en oxygène artérielle et/ou de la vitesse de flux sanguin et/ou de la quantité de sang transportée et/ou de la température du tissu, dans lequel un capteur optique (S) est posé sur le tissu, le tissu est éclairé par le capteur (S) avec la lumière d'une source de lumière blanche (W) et d'une source de lumière laser (L), la lumière renvoyée par le tissu est détectée, et les fractions renvoyées de la lumière blanche sont analysées de façon spectroscopique et/ou spectrométrique, et au moyen de la fraction renvoyée de la lumière laser, une analyse Doppler laser est effectuée.

**17.** Procédé selon la revendication 16, **caractérisé en ce qu'**en plus, la température du tissu est mesurée et analysée.

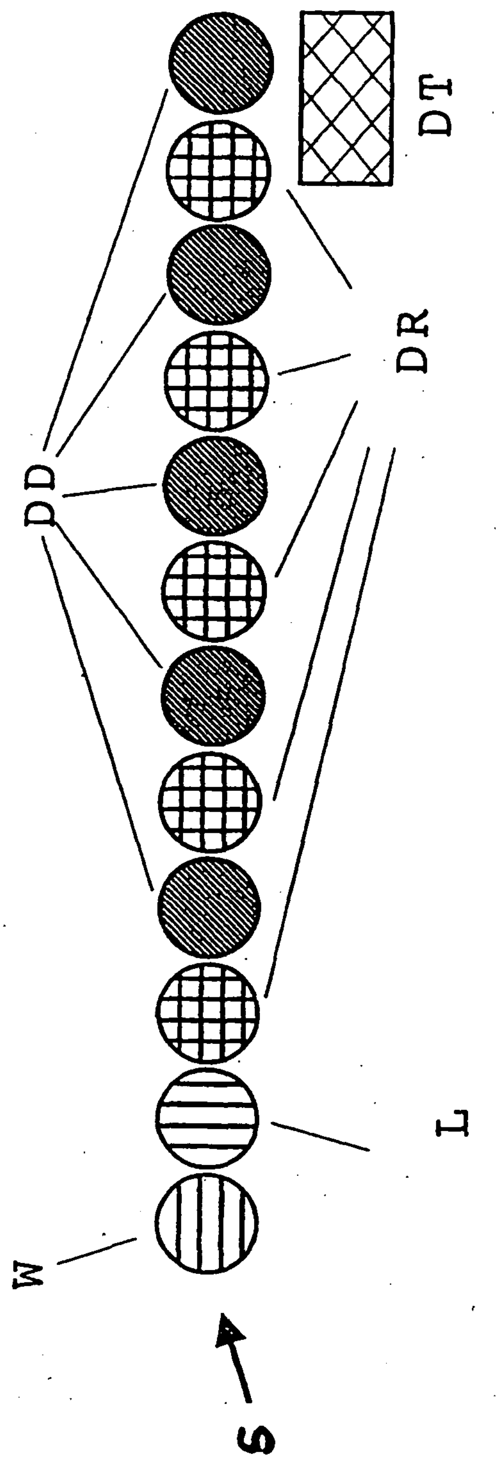


Fig. 1

Fig. 3

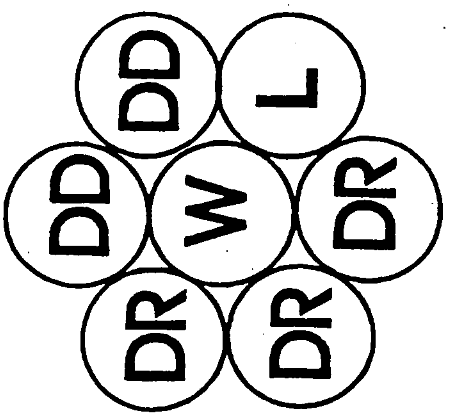


Fig. 4

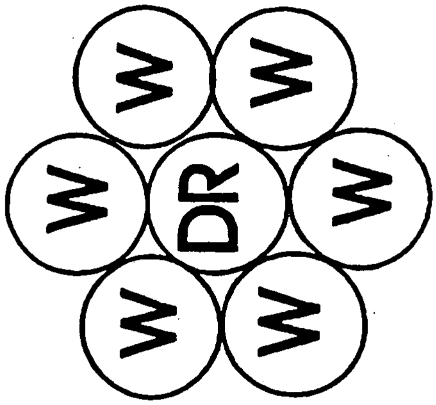


Fig. 2

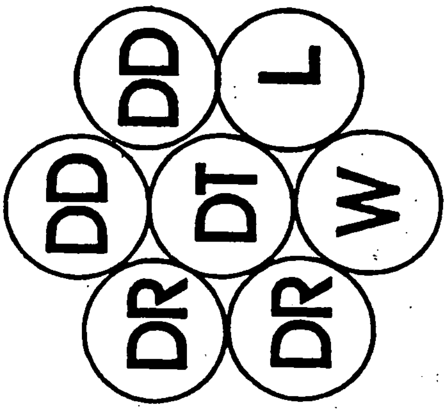

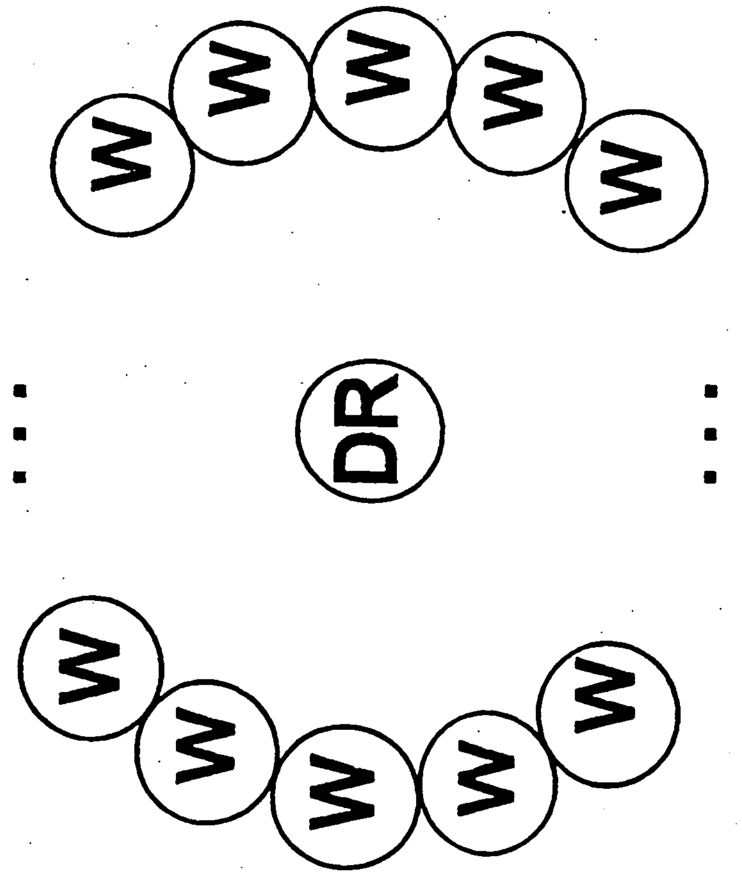


Fig. 5

Fig. 6

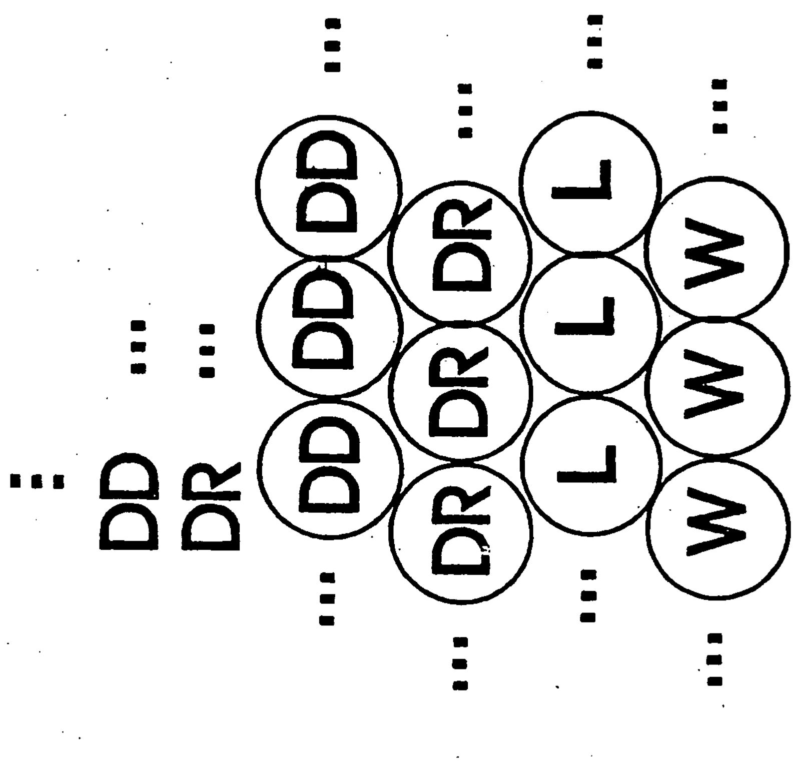

Fig.7

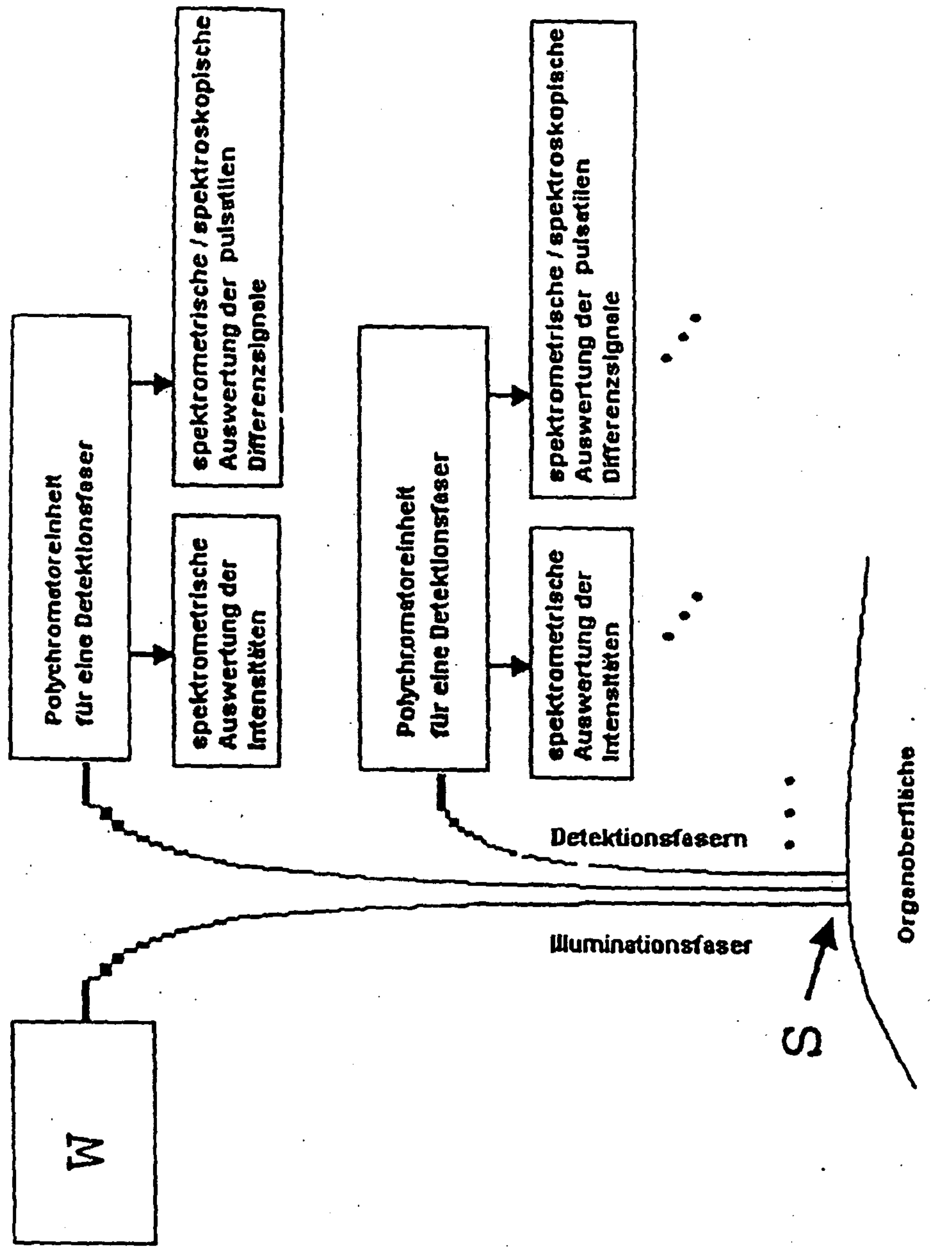
W
Polychromatoreinheit für eine Detektionsfaser
spektrometrische Auswertung der Intensitäten
spektrometrische / spektroskopische Auswertung der pulsatilen Differenzsignale
Polychromatoreinheit für eine Detektionsfaser
spektrometrische Auswertung der Intensitäten
spektrometrische / spektroskopische Auswertung der pulsatilen Differenzsignale
Detektionsfasern
Illuminationsfaser
S
Organoberfläche

Fig. 8

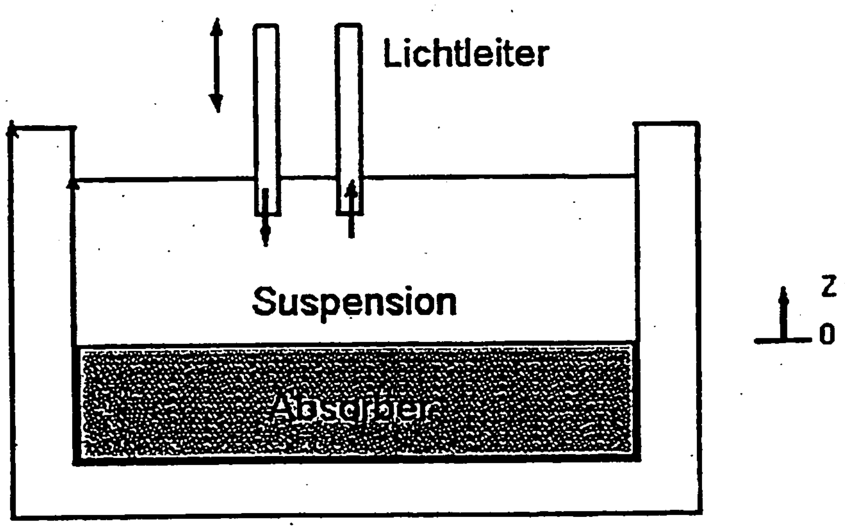

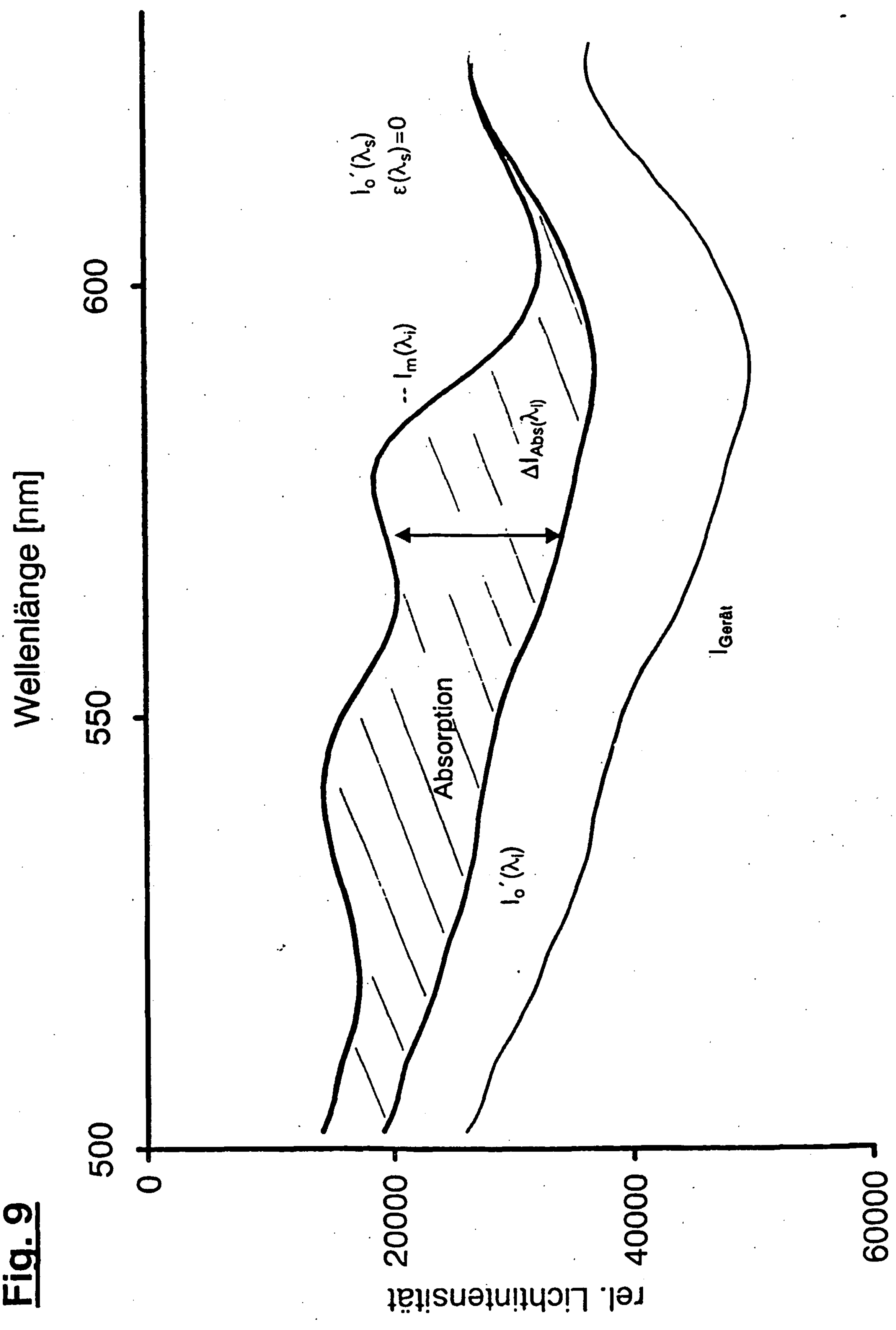
Wellenlänge [nm]
500
550
600
0
20000
40000
60000
rel. Lichtintensität
Absorption
-- $I_m(\lambda_i)$
$\Delta I_{Abs}(\lambda_i)$
$I_o'(\lambda_s)$
$\varepsilon(\lambda_s)=0$
$I_o'(\lambda_i)$
$I_{Gerät}$

Fig. 9

**Fig. 10**

**Hämoglobinspektren 0 - 100 % Sauerstoffsättigung**

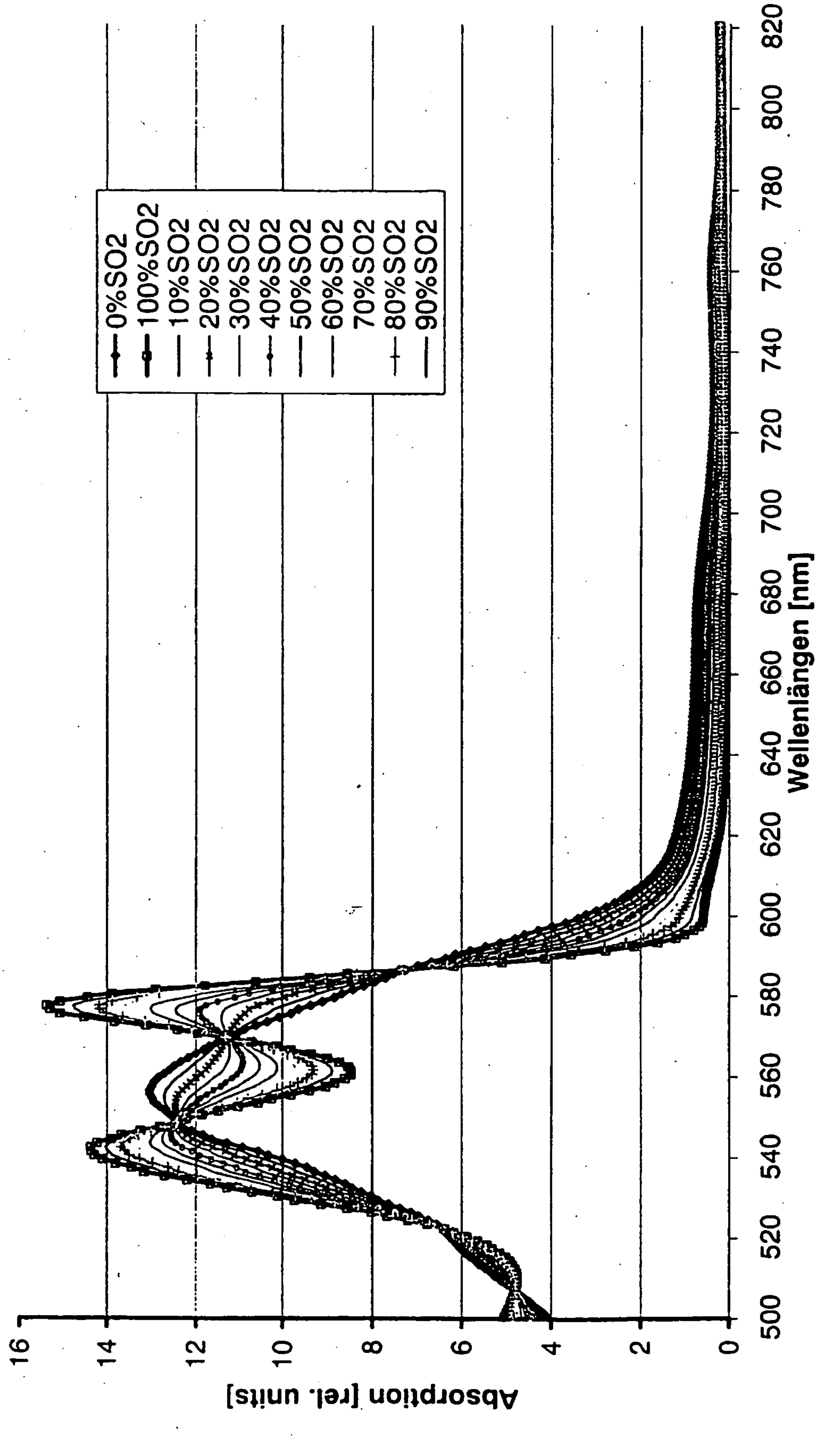

Fig. 11

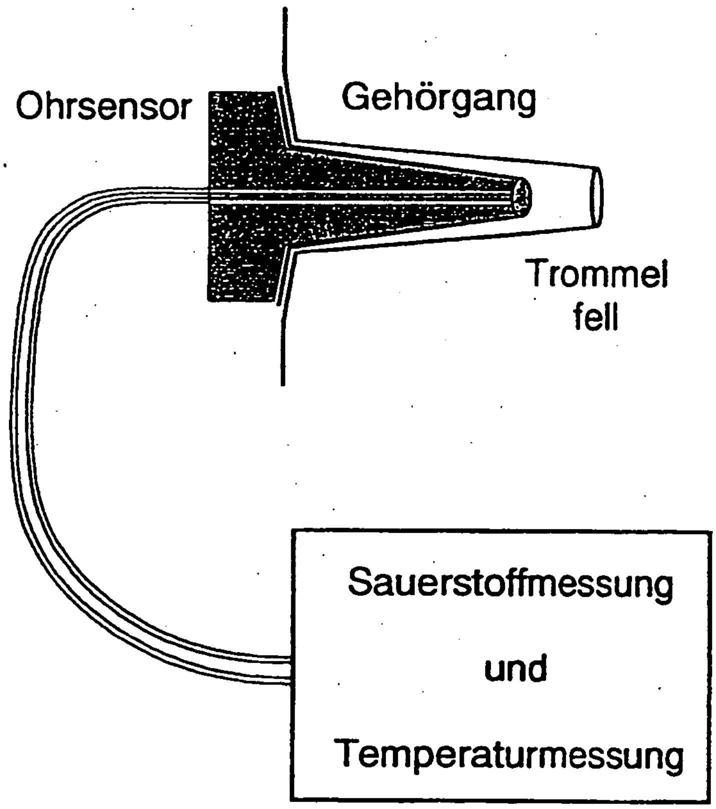
Ohrsensor
Gehörgang
Trommel
fell
Sauerstoffmessung
und
Temperaturmessung

Fig. 12

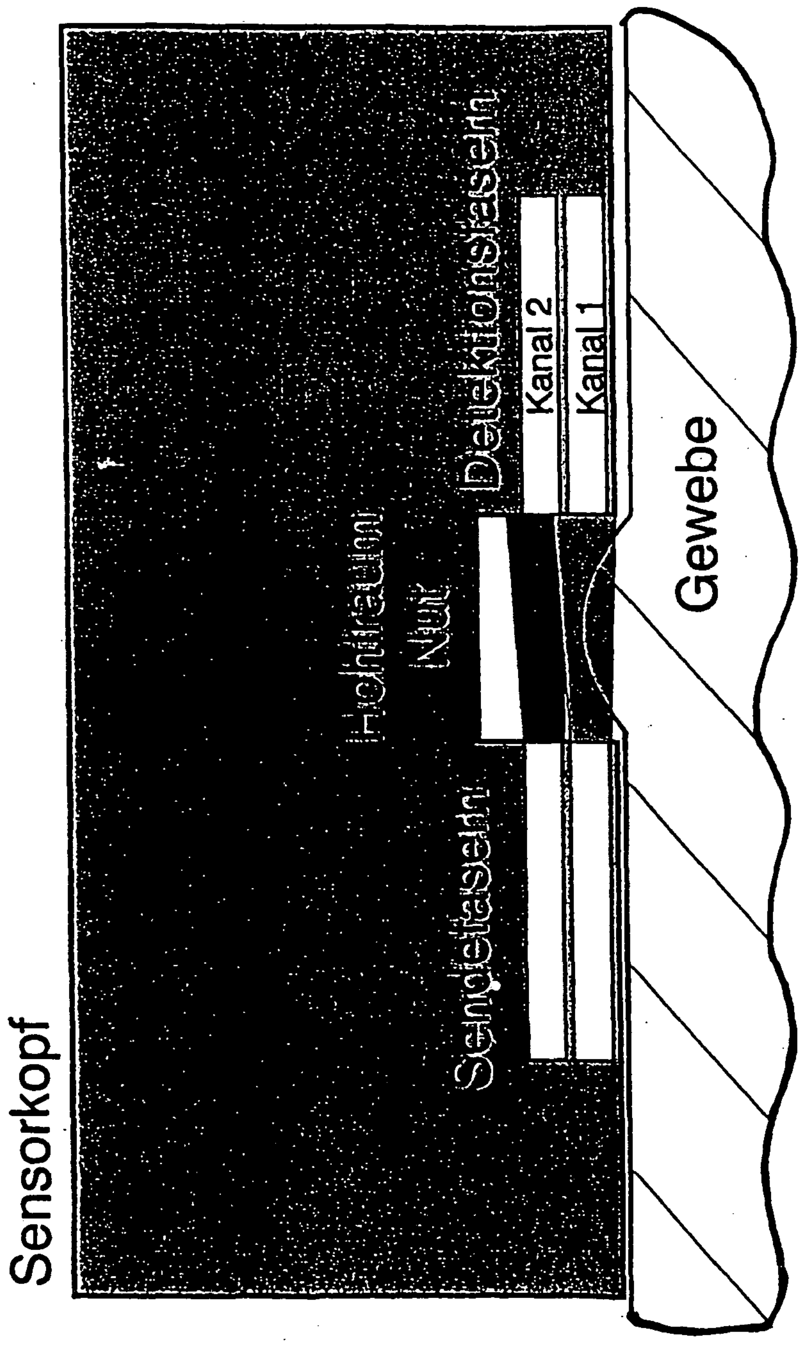
Sensorkopf
Sendefasern
Hohlraum
Nut
Detektionsfasern
Kanal 2
Kanal 1
Gewebe

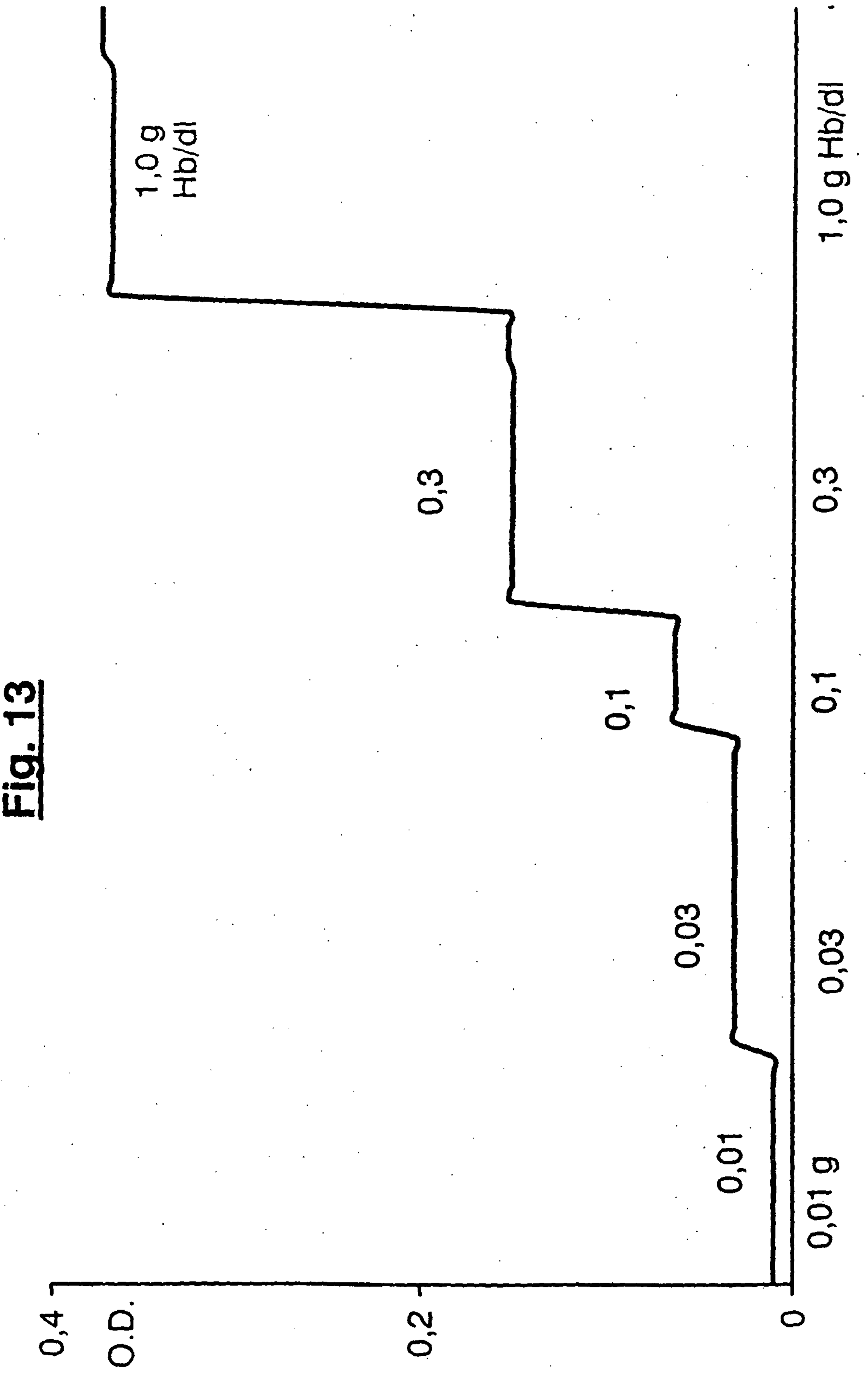


**Fig. 13**

Fig. 14

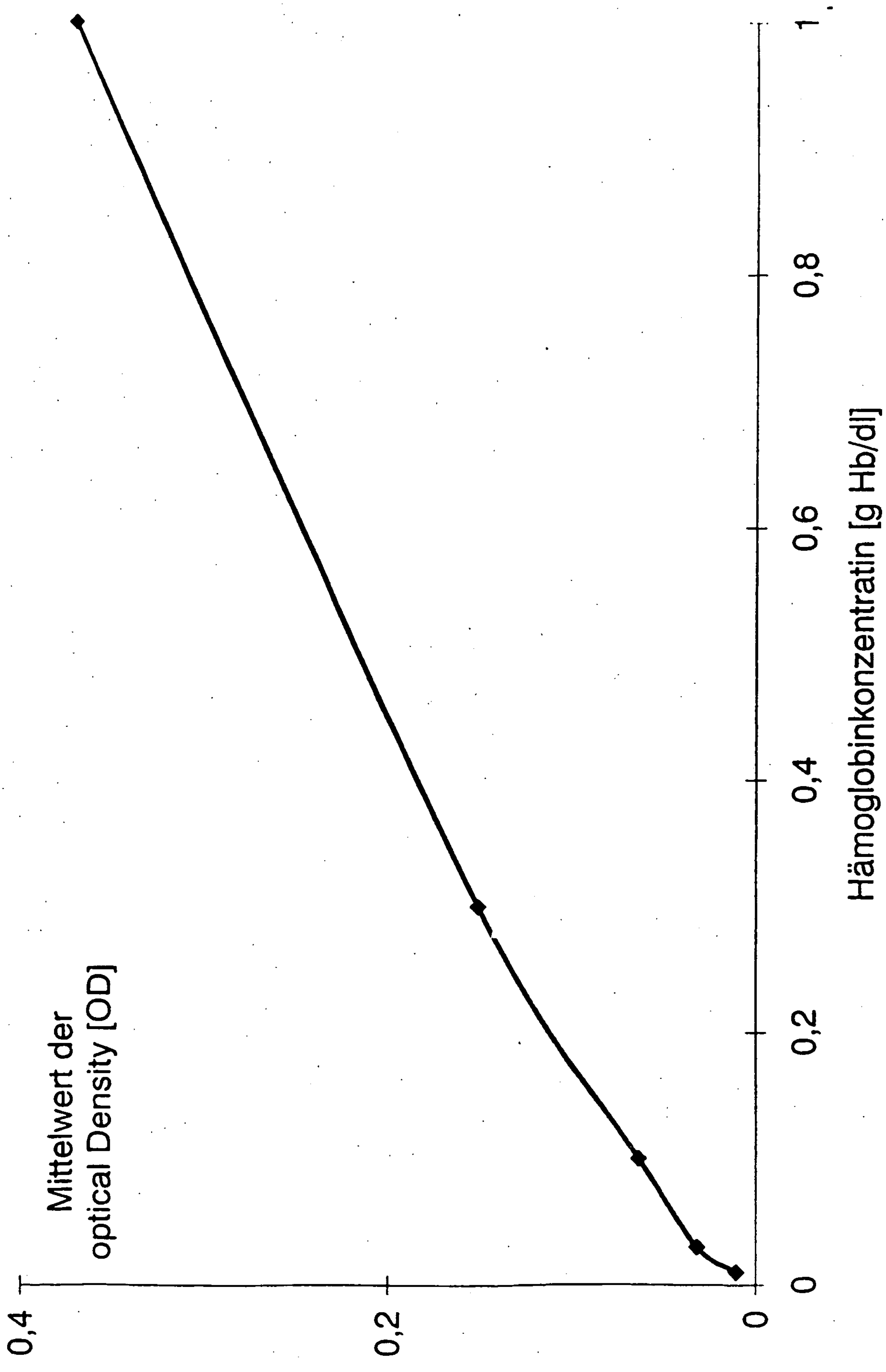
Mittelwert der
optical Density [OD]
0,4
0,2
0
0
0,2
0,4
0,6
0,8
1
Hämoglobinkonzentratin [g Hb/dl]

Fig. 15

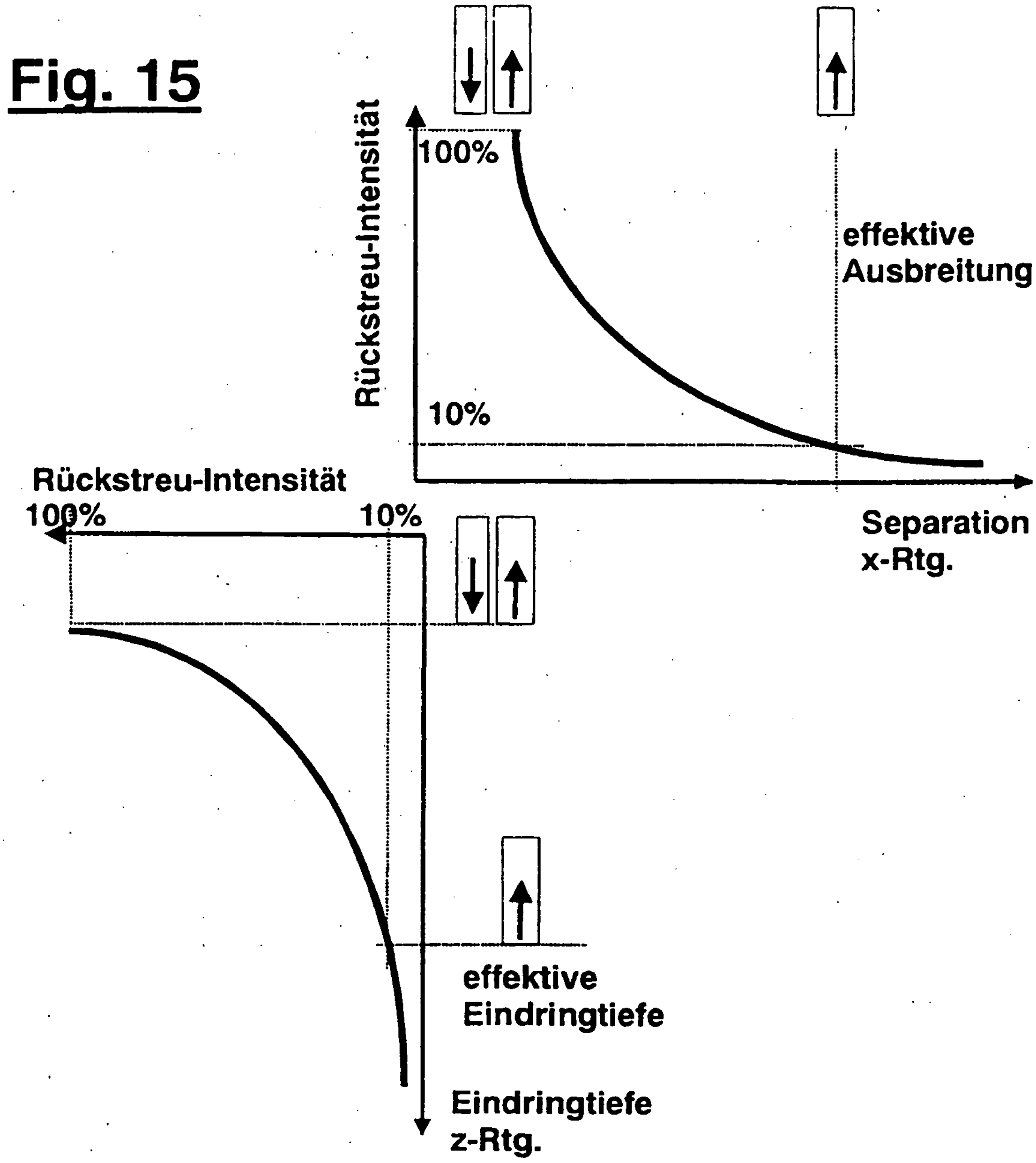
Rückstreu-Intensität
100%
10%
effektive
Ausbreitung
Separation
x-Rtg.
Rückstreu-Intensität
100%
10%
effektive
Eindringtiefe
Eindringtiefe
z-Rtg.

Fig. 16

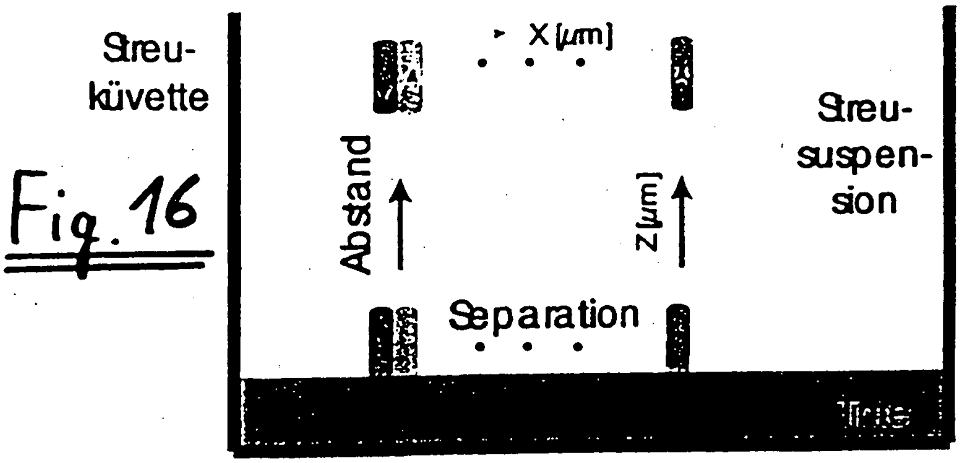


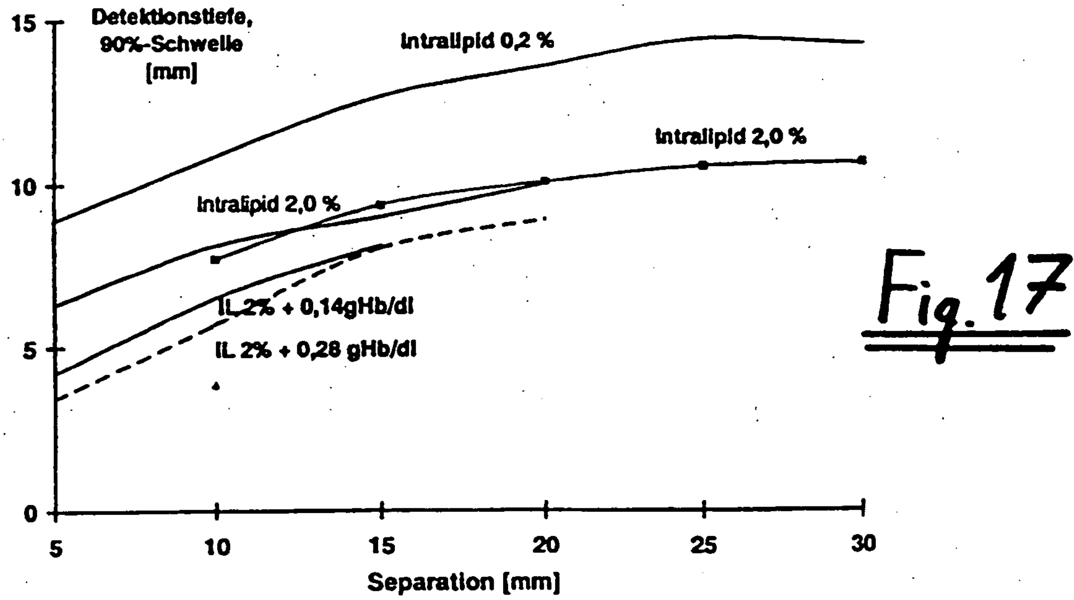


Fig. 17

Fig. 19

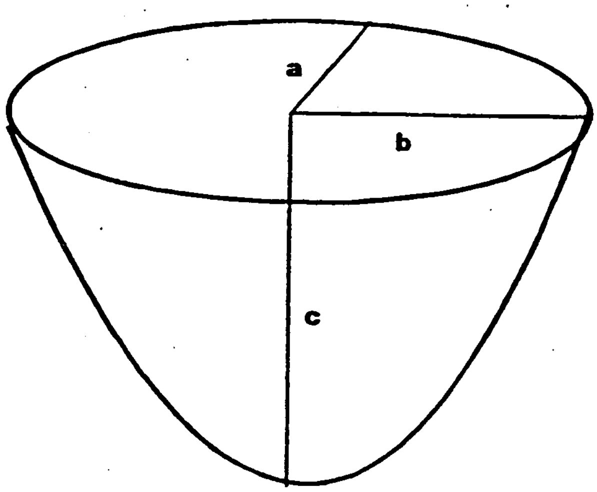

Fig. 18

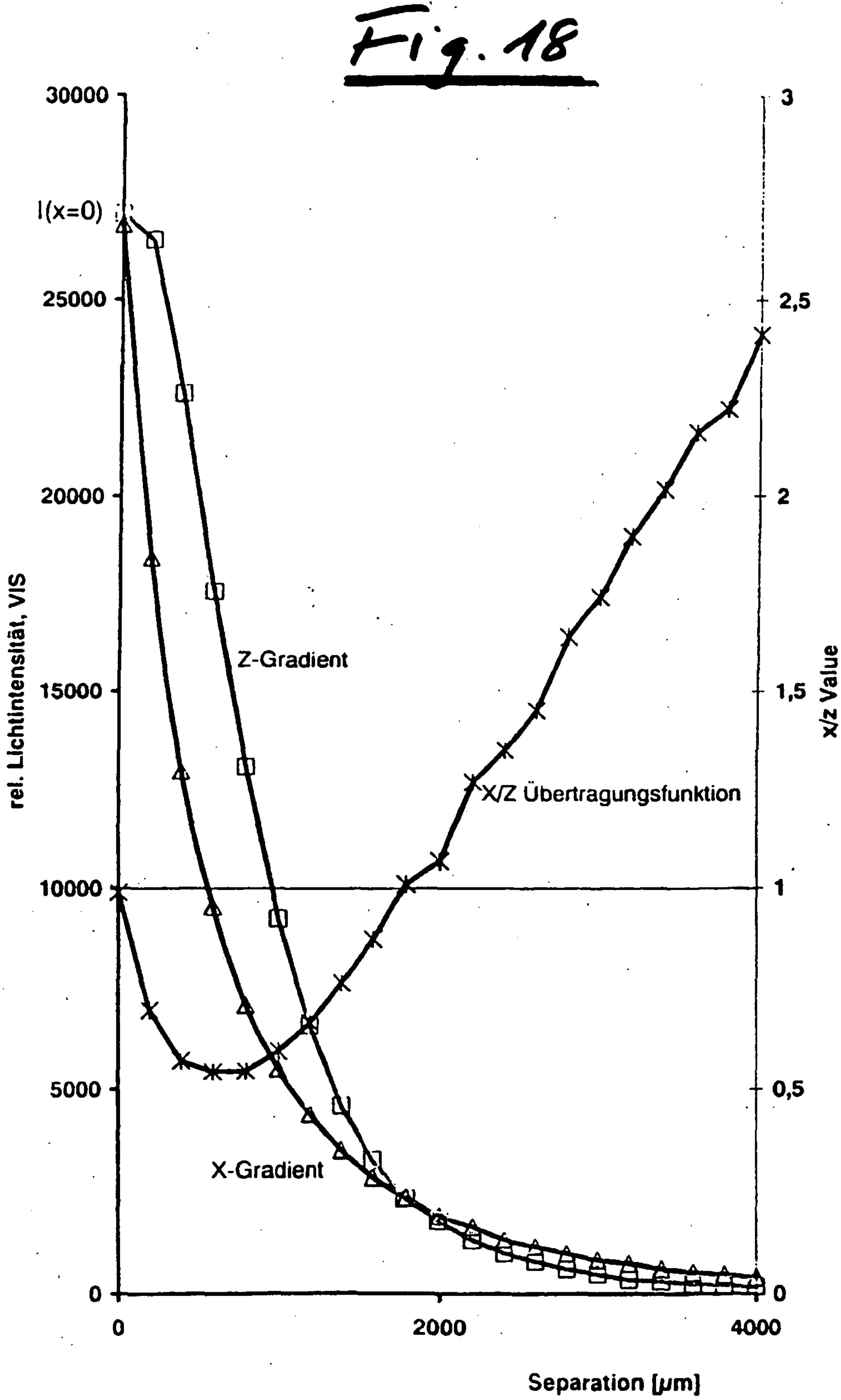
30000
I(x=0)
25000
20000
15000
10000
5000
0
rel. Lichtintensität, VIS
3
2,5
2
1,5
1
0,5
0
x/z Value
Z-Gradient
X/Z Übertragungsfunktion
X-Gradient
0
2000
4000
Separation [µm]

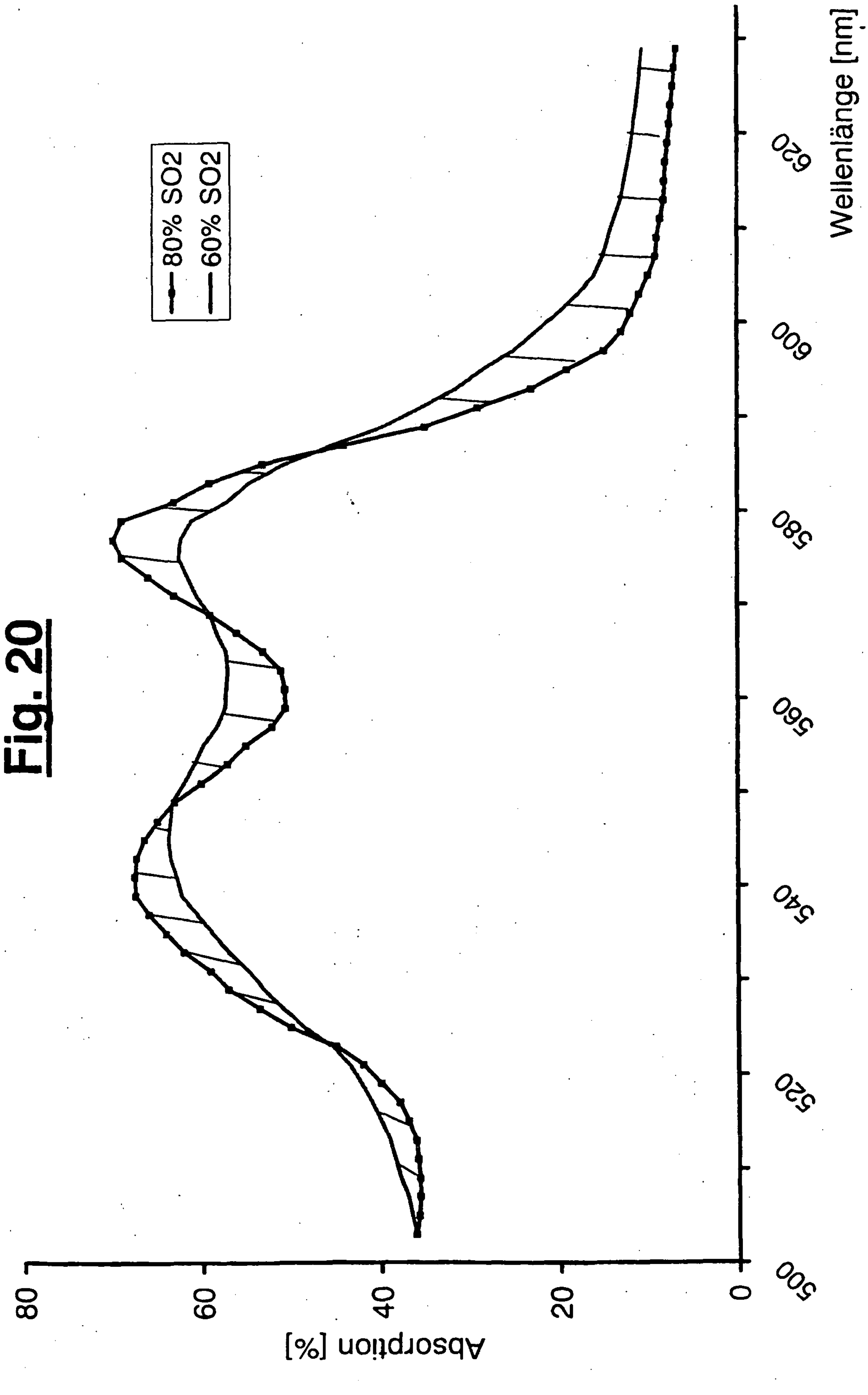

80% SO2
60% SO2
Absorption [%]
0
20
40
60
80
500
520
540
560
580
600
620
Wellenlänge [nm]


Fig. 20

## Fig. 21

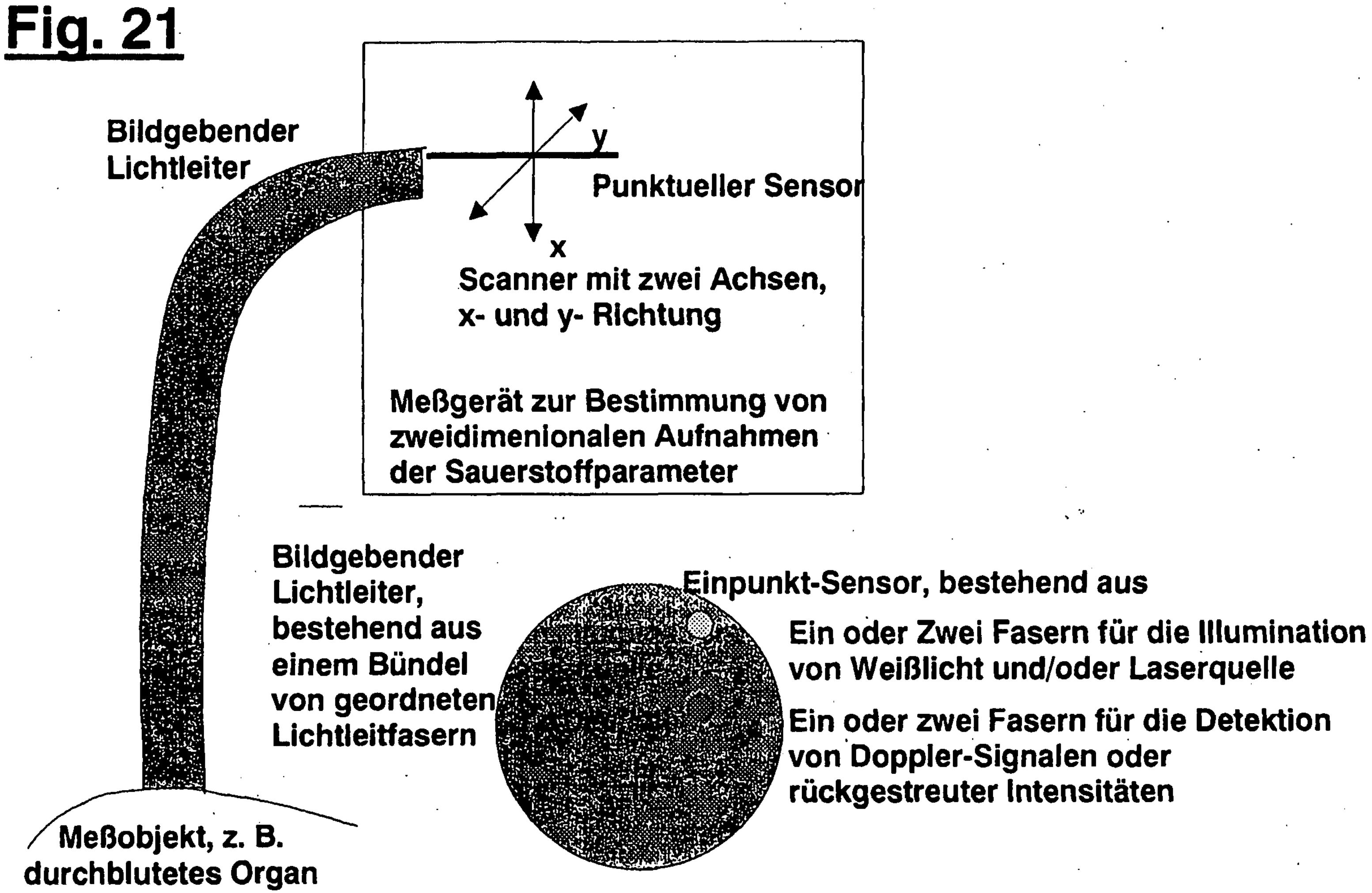
Bildgebender
Lichtleiter
y
x
Punktueller Sensor
Scanner mit zwei Achsen,
x- und y- Richtung
Meßgerät zur Bestimmung von
zweidimenionalen Aufnahmen
der Sauerstoffparameter
Bildgebender
Lichtleiter,
bestehend aus
einem Bündel
von geordneten
Lichtleitfasern
Einpunkt-Sensor, bestehend aus
Ein oder Zwei Fasern für die Illumination
von Weißlicht und/oder Laserquelle
Ein oder zwei Fasern für die Detektion
von Doppler-Signalen oder
rückgestreuter Intensitäten
Meßobjekt, z. B.
durchblutetes Organ

# Fig. 22

**$HbO_2$**

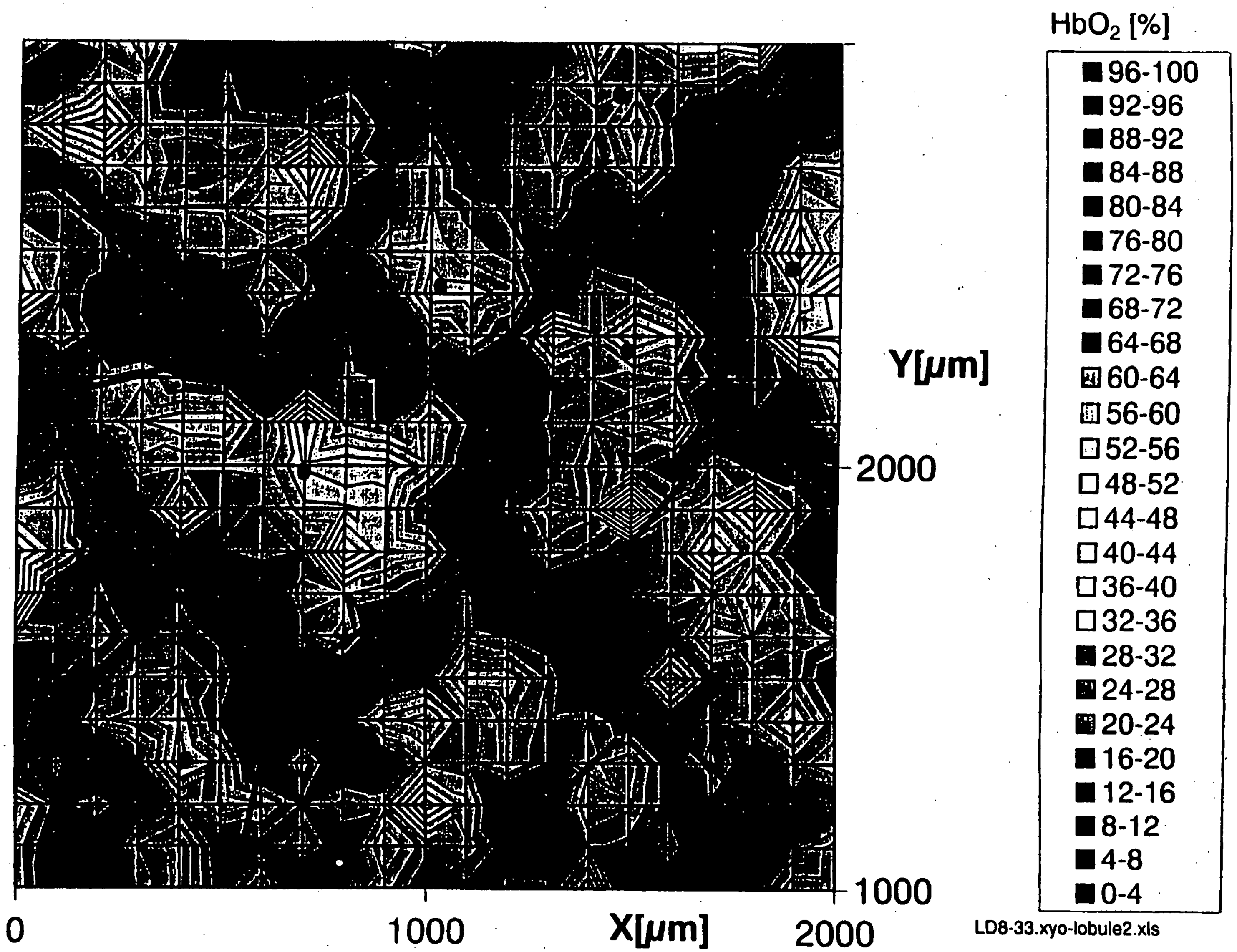
Y[µm]
2000
1000
0
1000
X[µm]
2000
HbO2 [%]
96-100
92-96
88-92
84-88
80-84
76-80
72-76
68-72
64-68
60-64
56-60
52-56
48-52
44-48
40-44
36-40
32-36
28-32
24-28
20-24
16-20
12-16
8-12
4-8
0-4
LD8-33.xyo-lobule2.xls

Fig. 23

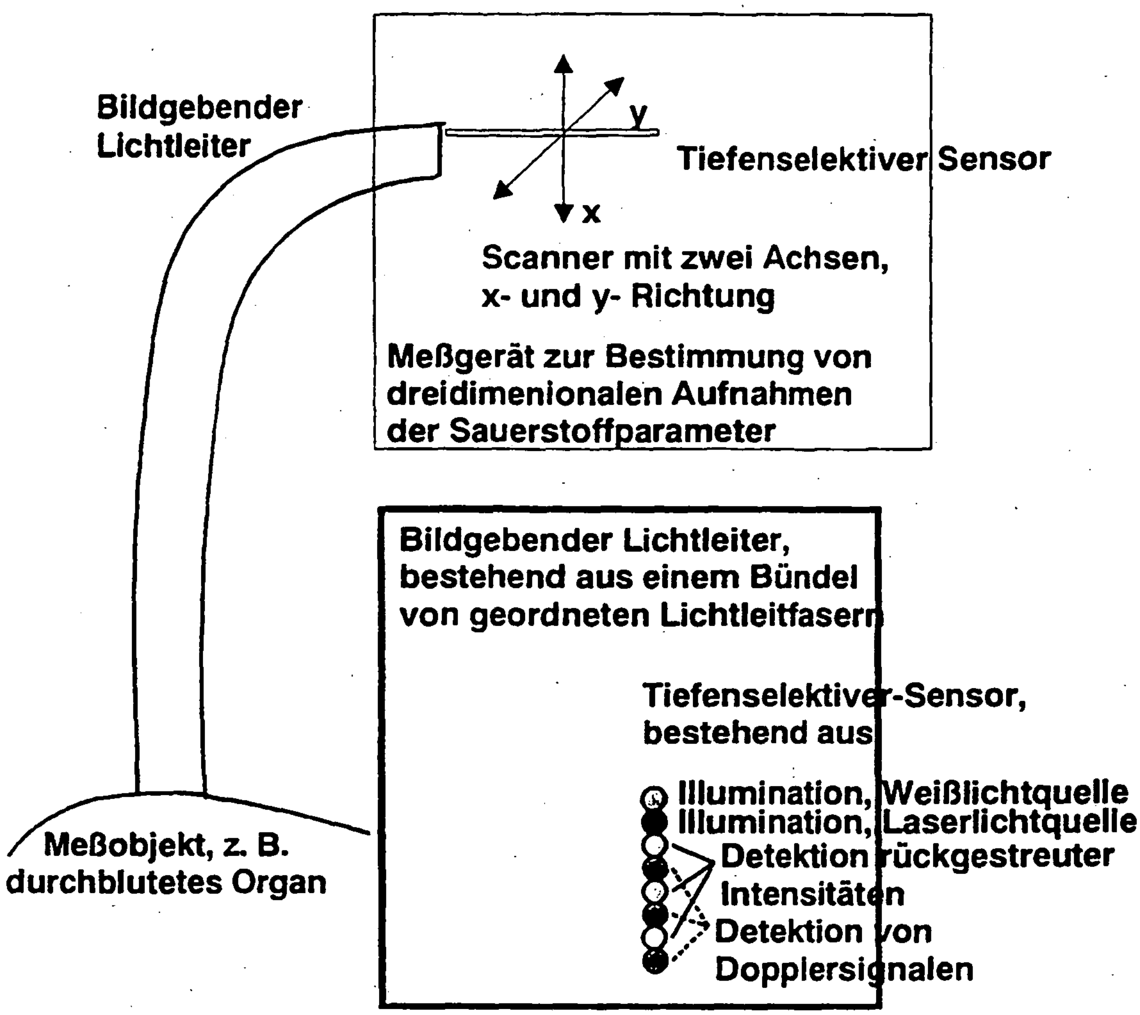
Bildgebender Lichtleiter
y
x
Tiefenselektiver Sensor
Scanner mit zwei Achsen, x- und y- Richtung
Meßgerät zur Bestimmung von dreidimenionalen Aufnahmen der Sauerstoffparameter
Bildgebender Lichtleiter, bestehend aus einem Bündel von geordneten Lichtleitfasern
Tiefenselektiver-Sensor, bestehend aus
Illumination, Weißlichtquelle
Illumination, Laserlichtquelle
Detektion rückgestreuter Intensitäten
Detektion von Dopplersignalen
Meßobjekt, z. B. durchblutetes Organ

Fig. 24

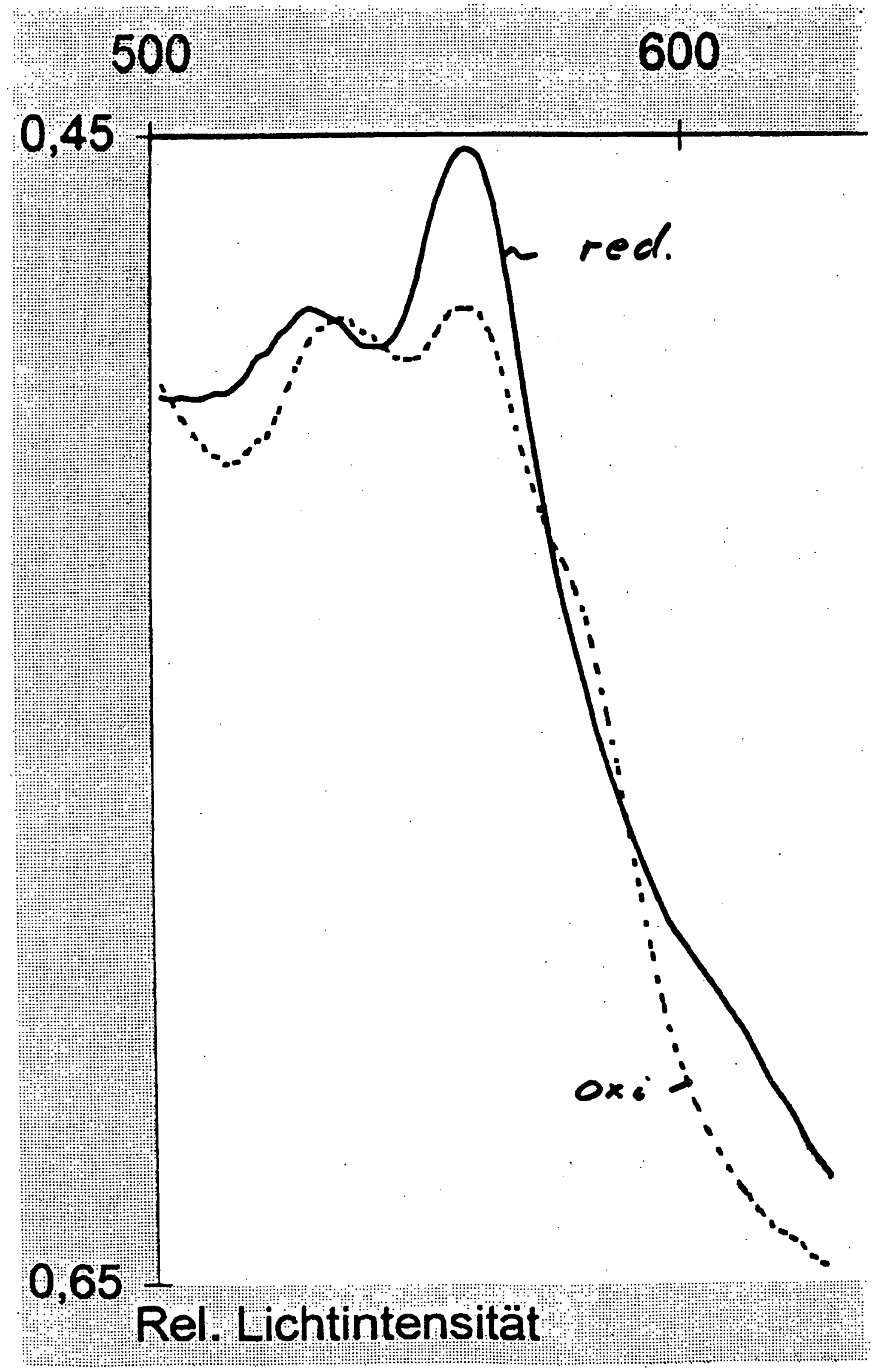

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0353619 A
- WO 9639927 A
- EP 771546 A2


### In der Beschreibung aufgeführte Nicht-Patentliteratur


- Quantitative optische Gewebemessungen am Herzen und an der Leber. *des Erfinders,* 24. Februar 1998, 2.4-1
- **A. KRUG.** *Dissertation, Uni Erlangen-Nürnberg,* 1998
- **W. DÜMMLER.** *Diss. Uni-Erlangen,* 1998
- **A. KRUG.** *Diss. Uni-Erlangen,* 1998
- **A. KRUG.** *Uni-Erlangen,* 1998
- **KRUG.** *Dissertation Uni Erlangen-Nürnberg,* 1998